(19) Europäisches Patentamt
European Patent Office
Office européen des brevets

(11) EP 3 740 191 B1

(12) EUROPEAN PATENT SPECIFICATION

(45) Date of publication and mention of the grant of the patent:
**28.04.2021 Bulletin 2021/17**

(21) Application number: **19701154.7**

(22) Date of filing: **09.01.2019**

(51) Int Cl.:
***A61K 9/00*** *(2006.01)* ***A61K 47/40*** *(2006.01)*
***A61K 31/454*** *(2006.01)* ***A61P 27/14*** *(2006.01)*

(86) International application number:
**PCT/EP2019/050433**

(87) International publication number:
**WO 2019/141563 (25.07.2019 Gazette 2019/30)**

(54) **OPHTHALMIC COMPOSITIONS COMPRISING BILASTINE, A BETA-CYCLODEXTRIN AND AT LEAST ONE GELLING AGENT**

OPHTHALMISCHE ZUSAMMENSETZUNGEN MIT BILASTIN, EINEM BETA-CYCLODEXTRIN UND MINDESTENS EINEM GELIERMITTEL

COMPOSITIONS OPHTALMIQUES COMPRENANT DE LA BILASTINE, UNE BÊTA-CYCLODEXTRINE ET AU MOINS UN AGENT GÉLIFIANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(30) Priority: **18.01.2018 EP 18382021**

(43) Date of publication of application:
**25.11.2020 Bulletin 2020/48**

(73) Proprietor: **FAES FARMA, S.A.**
**48940 Leioa - Vizcaya (ES)**

(72) Inventors:
- **HERNÁNDEZ HERRERO, Gonzalo**
**48940 Leioa - Vizcaya (ES)**
- **GONZALO GOROSTIZA, Ana**
**48940 Leioa - Vizcaya (ES)**
- **MORÁN POLADURA, Pablo**
**48940 Leioa - Vizcaya (ES)**
- **ZAZPE ARCE, Arturo**
**48940 Leioa - Vizcaya (ES)**
- **FERNÁNDEZ HERNANDO, Nieves**
**48940 Leioa - Vizcaya (ES)**
- **GONZÁLEZ GARCÍA, Tania**
**48940 Leioa - Vizcaya (ES)**
- **TATO CERDEIRAS, Paloma**
**48940 Leioa - Vizcaya (ES)**
- **OTERO ESPINAR, Francisco Javier**
**15782 Santiago de Compostela - A Coruña (ES)**
- **FERNÁNDEZ FERREIRO, Anxo**
**15782 Santiago de Compostela - A Coruña (ES)**
- **DÍAZ TOMÉ, Victoria**
**15782 Santiago de Compostela - A Coruña (ES)**

(74) Representative: **ABG Intellectual Property Law, S.L.**
**Avenida de Burgos, 16D**
**Edificio Euromor**
**28036 Madrid (ES)**

(56) References cited:
**WO-A2-2007/117971** **CN-A- 103 784 462**
**US-A- 5 419 898** **US-A1- 2005 239 745**
**US-A1- 2007 265 247**

- **JOEL R. GARBOW ET AL: "Structure, Dynamics, and Stability of [beta]-Cyclodextrin Inclusion Complexes of Aspartame and Neotame", JOURNAL OF AGRICULTURAL AND FOOD CHEMISTRY, vol. 49, no. 4, 1 April 2001 (2001-04-01), pages 2053-2060, XP055570834, US ISSN: 0021-8561, DOI: 10.1021/jf001122d**
- **YUEJIANG LIU ET AL: "In Situ Gelling Gelrite/Alginate Formulations as Vehicles for Ophthalmic Drug Delivery", AAPS PHARMSCITECH, vol. 11, no. 2, 31 March 2010 (2010-03-31) , pages 610-620, XP055570934, DOI: 10.1208/s12249-010-9413-0**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

Remarks:

The file contains technical information submitted after the application was filed and not included in this specification

## Description

### FIELD OF THE INVENTION

The present invention relates to aqueous pharmaceutical compositions containing a high concentration of bilastine suitable for once daily administration and their use as antihistamine and antiallergic ophthalmic pharmaceutical compositions.

### BACKGROUND

It has long been known that histamine plays a very important role in allergic-type diseases, such as allergic rhinitis, conjunctivitis, rhinoconjunctivitis, dermatitis, urticaria and asthma. Antihistaminic compounds acting at the Hi-receptor histamine level are useful for treating such conditions.

If the allergic symptoms are primarily ocular, then topical treatments appear to be preferred to systemic treatment. Topical agents are superior, with faster onset of action (within minutes) than systemic agents, and, thus, are readily able to retard the allergic response. In head-to-head comparisons, several studies utilising the conjunctival antigen challenge model demonstrated the superiority of topical agents over systemic antihistamines in the treatment of allergic conjunctivitis. Oral antihistamines can offer relief from other symptoms of allergy besides just ocular, but have a delayed onset of action when compared with topical ocular agents. Topical antihistaminic agents also have fewer adverse effects than systemic antihistamines because of the lower doses required to penetrate the conjunctivae and the negligible serum levels from topical use.

Despite the efficacy of the current available topical products to treat allergic conjunctivitis, patients experiencing incomplete symptom relief are likely to benefit from using a product that provides symptom relief over the course of an entire day with the convenience of once-daily dosing. Avoiding more frequent dosing is more convenient for patients since once-daily dosing is cost-effective, helps assure better patient compliance and more importantly, represents an improvement in their quality of life.

Documents EP 0818454 A1 and EP 0580541 A1 disclose benzimidazole compounds with selective $H_1$ antihistaminic activity and devoid of arrhythmogenic effects. Patent application EP 3040334 A1 also discloses benzimidazole compounds having potent selective $H_1$ antihistaminic activity, lacking activity on the central nervous system and on the cardiovascular system.

A particular compound with the above properties is 2-[4-(2-{4-[1-(2-Ethoxyethyl)-1H-benzimidazol-2-yl]-1-piperidinyl}ethyl)phenyl]-2-methylpropanoic acid, also known as bilastine, having formula:

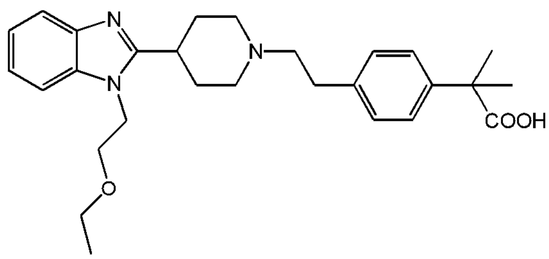

and developed by Faes Farma, Spain. Bilastine is a $H_1$ antagonist benzimidazole compound with no sedative side effects, no cardiotoxic effects, and no hepatic metabolism. In addition, bilastine has proved to be effective for the symptomatic treatment of allergic rhinoconjunctivitis and urticaria.

Bilastine was first disclosed in January of 1999 and, since then, many ophthalmic solutions comprising antihistaminic agents have been disclosed and are commercially available, for example EP 2709610 B1 discloses a topical ophthalmic solution containing a high concentration of olopatadine and the Food and Drug Administration (FDA) approved recently a new formulation of 0.77% olopatadine hydrochloride ophthalmic solution. However there is not a single enabling disclosure in the state of the art that provides an ophthalmic pharmaceutical composition containing a high concentration of bilastine suitable for once-daily administration.

Patent application WO 9413299 A1 discloses ophthalmic solutions of 1-(2-ethoxyethyl)-2-(4-methyl-1-homopiperazinyl)-benzimidazole, otherwise known as emedastine. Despite structurally similar to bilastine -emedastine is a benzimidazole derivative comprising an ethoxyethyl chain bound to the nitrogen atom of the imidazole ring-, the aqueous solubility of emedastine is much higher than that of bilastine which allows the provision of ophthalmic solutions comprising emedastine (at concentrations around 8 mg/mL) and with common excipients known to the skilled person such as EDTA, NaCl, hydroxypropyl methylcellulose (HPMC) and pH regulators NaOH/HCl and tris(hydroxymethyl)aminomethane.

The sole disclosures in the art which address the treatment of allergic symptoms in the eye by administering bilastine all refer to orally administered bilastine, highlighting the difficulty in providing ophthalmic solutions of this molecule. For example, Horak F, et al., Inflammation Research, 2010, (59) 391-398 or J Bartra et al., J. Investig. Allergol. Clin. Immunol., 2011, Vol. 21, Suppl.3: 24-33, only disclose bilastine tablets.

US2007265247 discloses a topical formulation comprising bilastine.

WO 03089425 A1 discloses that bilastine can be used for ophthalmic solutions but fails to provide any technical details on how to successfully prepare said solutions. WO 2007047253 A2 discloses methods of increasing the aqueous solubility of an antifungal azole using a hydroxybutenyl cyclodextrin. WO 2009003199 A1 discloses aqueous solution formulations comprising a corticosteroid, an antihistamine and sulfoalkyl ether cyclodextrin derivatives to nasal and ophthalmic tissues.

This problem is made evident when considering that, not only there is not a single enabling prior art disclosure that provides an ophthalmic pharmaceutical composition of high concentration of bilastine, but also the state of the art clearly points towards the use of olopatadine versus bilastine for the daily relief of allergic signs and symptoms. Beauregard C. et al., ARVO Annual Meeting Abstract May 2008 even teaches that bilastine does not equal the anti-histaminic potency or duration of action of olopatadine *in vivo.*

CN 103784462 A discloses an ophthalmic preparation comprising bilastine and steroids as the active ingredients formulated in combination with auxiliary substances such as methylcellulose, cyclodextrin, sorbitol and alginic acid that enable such composition to be administered to the eye for the treatment of conjunctivitis. However, it fails to disclose the beta-cyclodextrin as claimed.

WO 2007/117971 A2 relates to the treatment or prevention of ocular conditions with compositions of an H1 antagonist. This document refers to such topical preparation which has a longer duration of action and is advantageous because it may be instilled once daily. It fails to disclose bilastine.

US 2005/239745 A1 discloses topical ophthalmic formulations comprising ketotifen fumarate in combination with additional agents such as cyclodextrins, gelling agents (hyaluronic acid), tonicity agents (glycerin, sorbitol, mannitol) and polymers (cellulose and polyethylene glycol) that provide a comfortable preparation when instilled in the eye for treating ocular allergy. However, it fails to disclose bilastine.

US 5 419 898 A relates to an anti-allergic composition for ophthalmic or nasal use comprising cetirizine and cyclodextrin compounds, which improve the stability of the drug. Example 6 shows the results from a stability test of compositions comprising cyclodextrin. However, it fails to disclose bilastine.

Joel R. Garbow et al., J Agric Food Chem., vol 49(4), 2053-2060, 2001, describes that the stability of the aspartame is enhanced in the presence of beta-cyclodextrin. However, it fails to disclose the composition as presently claimed.

Yuejiang Liu et al., AAPS PHARMSCITECH, vol. 11, no. 2, 610-620, 2010, describes the results from pharmacological studies of a solution comprising gelling agents (alginate and deacetylated gellan gum). However, it fails to disclose the composition as presently claimed.

Therefore, there is a need in the art to provide an ophthalmic solution suitable for once-daily administration that includes high concentrations of said benzimidazole compounds.

## BRIEF DESCRIPTION OF THE INVENTION

The inventors of the present invention have surprisingly found that the ophthalmic formulations of the invention, which comprise bilastine result in the unexpected increase in the duration of the compound's ocular efficacy. These findings are particularly surprising when taking into account that such increase in efficacy is not observed in preclinical studies involving bilastine and the comparative antihistamine compounds olopatadine and azelastine. The present invention thus provides for the first time a once-daily ophthalmic formulation comprising bilastine.

Consequently, in a first aspect, the invention provides an ophthalmic pharmaceutical composition comprising:

a) at least 0.4% w/v but no more than 1.0% w/v of bilastine, or a pharmaceutically acceptable salt or solvate thereof, wherein the bilastine or salt or solvate thereof is completely dissolved in the aqueous ophthalmic pharmaceutical composition;

b) at least one β-cyclodextrin, selected from the group consisting of unmodified β-cyclodextrin, $C_1$-$C_6$ alkyl-β-cyclodextrin, $C_1$-$C_6$ hydroxyalkyl β-cyclodextrin, $C_1$-$C_6$ carboxyalkyl-β-cyclodextrin, carbonyl-β-cyclodextrin, $C_2$-$C_6$ sulfoalkylether β-cyclodextrin and mixtures thereof; and

c) at least one pharmaceutically acceptable water-soluble gelling agent or an acceptable salt thereof, selected from the group consisting of hyaluronic acid, Gellan gum and mixtures thereof;

and wherein the pH value of the composition is comprised between 4 and 9, both lower and upper limits of the range included.

In a second aspect, this invention refers to the aqueous ophthalmic pharmaceutical composition mentioned above for use in the treatment and/or prevention of a disorder or disease susceptible to amelioration by antagonism of $H_1$ histamine receptor, selected from an ocular allergic disorder or an allergic disease.

Another aspect of this invention refers to the aqueous ophthalmic pharmaceutical composition mentioned above and a further pharmaceutically acceptable carrier, adjuvant or vehicle.

These aspects and preferred embodiments thereof are also additionally described further down in the description and defined in the claims.

## BRIEF DESCRIPTION OF THE FIGURES

Figure 1 shows the stability of bilastine solutions at a concentration over 6.5 mg/mL in the presence of 9 % w/v of five β-cyclodextrins in different pH media at to.

Figure 2 shows the stability of bilastine solutions at a concentration over 6.5 mg/mL in the presence of 9 % w/v of five β-cyclodextrins in different pH media at $t_1$.

Figure 3 shows the Ocular Average Retention Times (ART) for compositions of the invention comprising bilastine 0.4% w/v and HPB 9% w/v. AH is Sodium hyaluronate, GG is Gellan gum and MC is methylcellulose.

Figure 4 shows the effect of the ophthalmic formulations of the invention on histamine-induced conjunctivitis in guinea pig.

Figure 5 shows treatment differences represented by mean ocular itching score calculated as Active-Vehicle using least square means (LSMeans), measured for three bilastine concentrations (bilastine 0.2% w/v, bilastine 0.4% w/v and bilastine 0.6% w/v) at different time (minutes) post-Conjunctival Allergen Challenge (CAC®): at baseline, 16 hours, 8 hours, and 15 minutes.

Figure 6 shows mean Ocular Itching Scores (0-4 scale) measured at different time (minutes) post-CAC®: at baseline, 16 hours, 8 hours and 15 minutes.

## DETAILED DESCRIPTION OF THE INVENTION

The inventors have surprisingly found that the combination of bilastine with at least a β-cyclodextrin and at least one pharmaceutically acceptable water-soluble gelling agent or an acceptable salt thereof, is optimal for ophthalmic applications, showing excellent prolonged action in the eye which allows the provision of a once-daily ophthalmic formulation comprising bilastine. Surprisingly, these results were not observable in preclinical trials, as shown in the Examples referring to preclinical and clinical studies.

Consequently, in a first aspect, the invention provides an ophthalmic pharmaceutical composition comprising:

a) at least 0.4% w/v but no more than 1.0% w/v of bilastine, or a pharmaceutically acceptable salt or solvate thereof, wherein the bilastine or salt or solvate thereof is completely dissolved in the aqueous ophthalmic pharmaceutical composition;

b) at least one β-cyclodextrin, selected from the group consisting of unmodified β-cyclodextrin, $C_1$-$C_6$ alkyl-β-cyclodextrin, $C_1$-$C_6$ hydroxyalkyl β-cyclodextrin, $C_1$-$C_6$ carboxyalkyl-β-cyclodextrin, carbonyl-β-cyclodextrin, $C_2$-$C_6$ sulfoalkylether β-cyclodextrin and mixtures thereof; and

c) at least one pharmaceutically acceptable water-soluble gelling agent or an acceptable salt thereof, selected from the group consisting of hyaluronic acid, Gellan gum and mixtures thereof;

and wherein the pH value of the composition is comprised between 4 and 9, both lower and upper limits of the range included.

*Bilastine*

The aqueous ophthalmic pharmaceutical composition of the invention comprises bilastine, of formula,

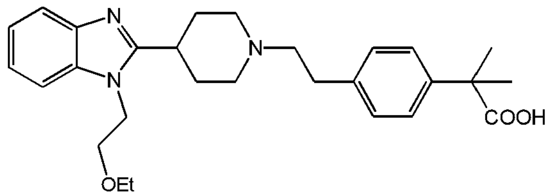

or a pharmaceutically acceptable salt or solvate thereof. This compound is the 2-[4-(2-{4-[1-(2-Ethoxyethyl)-1H-benzimidazol-2-yl]-1-piperidinyl}ethyl)phenyl]-2-methylpropanoic acid, also known as bilastine. The synthesis of bilastine has been described in documents EP 0818454 A1, EP 0580541 A1 and EP 3040334 A1.

Bilastine may be in the form of a salt or solvate, preferably pharmaceutically acceptable salts or solvates.

The invention also provides "salts" of the compounds described herein. By way of illustration, said salts can be acid addition salts, base addition salts or metal salts, and can be synthesized from the parent compounds containing a basic or acid moiety by means of conventional chemical processes known by the persons skilled in the art. Such salts are generally prepared, for example, by reacting the free acid or base forms of said compounds with a stoichiometric amount of the suitable base or acid in water or in an organic solvent or in a mixture of the two. Non-aqueous media such as ether, ethyl acetate, ethanol, acetone, isopropanol or acetonitrile are generally preferred. Illustrative examples of acid addition salts include inorganic acid addition salts such as, for example, hydrochloride, hydrobromide, hydroiodide, sulfate, nitrate, phosphate, etc., organic acid addition salts such as, for example, acetate, maleate, fumarate, citrate, oxalate, succinate, tartrate, malate, mandelate, methanesulfonate, *p*-toluenesulfonate, camphorsulfonate, etc. Illustrative examples of base addition salts include inorganic base salts such as, for example, ammonium salts and organic base salts such as, for example, ethylenediamine, ethanolamine, *N,N*-dialkylenethanolamine, triethanolamine, glutamine, amino acid basic salts, etc. Illustrative examples of metal salts include, for example, sodium, potassium, calcium, magnesium, aluminium and lithium salts.

The term "solvate" according to this invention is to be understood as meaning any form of the active compound according to the invention which has another molecule (most likely a polar solvent) attached to it via non-covalent bonding. Examples of solvates include hydrates and alcoholates. Solvation methods are generally known in the state of the art.

The compounds of the invention are also meant to include compounds which differ only in the presence of one or more isotopically enriched atoms. For example, compounds having the present structures except for the replacement of a hydrogen by a deuterium or tritium, or the replacement of a carbon by a $^{13}C$- or $^{14}C$-enriched carbon or a nitrogen by $^{15}N$-enriched nitrogen are within the scope of this invention.

A concentration of at least 0.4% w/v means that there are more than 4 mg of bilastine completely dissolved, i.e, without any significant signs of precipitation, in just 1 mL of water. In the present invention, all the percentages are in w/v units unless otherwise stated. In another embodiment, bilastine is completely dissolved in the pharmaceutical composition of the invention at a concentration of at least 0.6% w/v, preferably at least 0.7% w/v. In another embodiment, bilastine is completely dissolved in the pharmaceutical composition of the invention at a concentration of at least 0.6% w/v but no more than 1.0% w/v. In a particular embodiment, the pharmaceutical composition comprises at least 0.4% w/v, at least 0.6% w/v, at least 0.7% w/v, at least 0.8% w/v, at least 0.9% w/v, or at least 0.6% but no more than 1.0% w/v of bilastine, wherein bilastine is completely dissolved. In another particular embodiment, the pharmaceutical composition comprises at least 0.4% w/v, at least 0.6% w/v, at least 0.7% w/v, at least 0.8% w/v, at least 0.9% w/v, or at least 0.6% but no more than 1.0% w/v of bilastine, wherein bilastine is completely dissolved. In a preferred embodiment, bilastine is completely dissolved in the pharmaceutical composition of the invention at a concentration of at least 0.6% w/v but no more than 1.0% w/v.

*Cyclodextrins*

In the context of the present invention, a cyclodextrin (CD) is a cyclic structure composed of 5 or more β-D-glucopyranose units linked at the 1,4 positions, typically having 6 (a-cyclodextrin), 7 (β-cyclodextrin), 8 (y-cyclodextrin) or 9 (δ-cyclodextrin) sugar units in one cyclodextrin molecule.

The MS value (average molar degree of substitution) is the average number of moles of substituent groups per glucopyranose mol. For example, in the case of β-cyclodextrins, the average number of substituents per β-cyclodextrin core can be calculated by multiplying the MS value by 7 (the β-cyclodextrin comprises 7 sugar units per cyclodextrin molecule). An unambiguous notation for this value is the DS (degree of substitution).

Both amorphous and crystalline cyclodextrins are within the scope of the present application. As used herein the term "cyclodextrin" may refer to a cyclodextrin or a cyclodextrin derivative. Cyclodextrins are commercially available or may be synthesized by methods well-known in the art. Examples of cyclodextrins include, but are not limited to,

modified or unmodified α-, β-, γ- and δ-cyclodextrins. The cyclodextrins of the present invention are β-cyclodextrins. Derivatives of cyclodextrins and particularly of the β-cyclodextrins of the invention include those wherein some or all of the OH groups are converted to OR groups. Said derivatives include those with $C_{1-6}$ alkyl groups such as e.g. methylated, ethylated, propylated and butylated cyclodextrins, wherein R is a methyl, ethyl, propyl or butyl group; those with hydroxyalkyl substituted groups such as e.g. hydroxypropyl cyclodextrins or hydroxyethyl cyclodextrins, wherein R is a $-CH_2CH(OH)CH_3$ or a $CH_2CH_2OH$ group; branched cyclodextrins such as maltose-bonded cyclodextrins; cationic cyclodextrins; quaternary ammonium; anionic cyclodextrins such as carboxymethyl cyclodextrins, cyclodextrin sulfates and cyclodextrin succinates; amphoteric cyclodextrins such as carboxymethyl/quaternary ammonium cyclodextrins. Other specific modifications include one or more hydroxyalkyl ether (e.g. R is $C_{1-6}$ alkylenehydroxy) moieties; one or more sulfoalkyl ether (e.g. R is $C_{2-6}$ alkylene$SO_3^-$) moieties; carboxyalkyl (e.g. R is $C(O)C_{1-6}$alkyl) moieties; substituted phenoxy moieties; tryptofan moieties; or mixtures thereof. The total number of OR groups per cyclodextrin molecule is defined as the degree of substitution/modification.

In the present invention, the cyclodextrin of the ophthalmic pharmaceutical composition is a β-cyclodextrin. In a preferred embodiment, the β-cyclodextrin is selected from the group consisting of $C_1$-$C_6$ alkyl-β-cyclodextrin, $C_1$-$C_6$ hydroxyalkyl β-cyclodextrin, $C_1$-$C_6$ carboxyalkyl-β-cyclodextrin, carbonyl-β-cyclodextrin, $C_2$-$C_6$ sulfoalkylether β-cyclodextrin and mixtures thereof.

In one embodiment the β-cyclodextrin is an $C_1$-$C_6$ alkyl-β-cyclodextrin. Preferred $C_1$-$C_6$ alkyl-β-cyclodextrins include methyl-β-cyclodextrin; dimethyl-β-cyclodextrin; trimethyl-β-cyclodextrin; ethyl-β-cyclodextrin; diethyl-β-cyclodextrin; propyl-β-cyclodextrin; and butyl-β-cyclodextrin. In a more preferred embodiment the β-cyclodextrin is selected from the group consisting of methyl-β-cyclodextrin or dimethyl-β-cyclodextrin. $C_1$-$C_6$ alkyl β-cyclodextrin derivatives preferably have a degree of substitution/modification of from about 1 to about 18, from about 3 to about 16, from about 4 to about 14, from about 4 to about 12.6, and more preferably from about 4 to 6. In a particular embodiment the β-cyclodextrin is not an alkenyl-β-cyclodextrin, particularly the β-cyclodextrin is not hydroxybutenyl-β-cyclodextrin.

In another embodiment the cyclodextrin is a $C_1$-$C_6$ hydroxyalkyl-β-cyclodextrin. Preferred $C_1$-$C_6$ hydroxyalkyl-β-cyclodextrins include hydroxyethyl-β-cyclodextrin; hydroxypropyl-β-cyclodextrin (which is equivalent to 2-hydroxypropyl-β-cyclodextrin) and 2-hydroxybutyl-β-cyclodextrin. In a more preferred embodiment the cyclodextrin is hydroxypropyl-β-cyclodextrin (HPBCD or HP-β-CD).

$C_1$-$C_6$ hydroxyalkyl cyclodextrin derivatives, and particularly the hydroxypropyl-β-cyclodextrin, preferably have a degree of substitution/modification of from about 1 to about 14, more preferably from about 4 to about 8.

In a further embodiment the cyclodextrin is a $C_1$-$C_6$ carboxyalkyl-β-cyclodextrin. Preferred $C_1$-$C_6$ carboxyalkyl-β-cyclodextrins for use herein include carboxymethyl-β-cyclodextrin and (2-carboxyethyl)-β-cyclodextrin.

In a further embodiment the cyclodextrin is a $C_2$-$C_6$ sulfoalkylether β-cydodextrin. A preferred $C_2$-$C_6$ sulfoalkylether-β-cyclodextrin for use herein is sulfobutylether-β-cyclodextrin.

$C_2$-$C_6$ sulfoalkylether-β-cyclodextrin derivatives preferably have a degree of substitution/modification from about 1 to about 14, preferably from about 1 to about 7.

In a preferred embodiment the cyclodextrin is a β-cyclodextrin selected from the group consisting of $C_1$-$C_6$ alkyl-β-cyclodextrin, $C_1$-$C_6$ hydroxyalkyl β-cyclodextrin, $C_1$-$C_6$ carboxyalkyl-β-cyclodextrin, $C_2$-$C_6$ sulfoalkylether β-cyclodextrin and mixtures thereof.

In another preferred embodiment, the cyclodextrin is a β-cyclodextrin selected from the group consisting of $C_1$-$C_6$ hydroxyalkyl β-cyclodextrin, $C_1$-$C_6$ carboxyalkyl-β-cyclodextrin, $C_2$-$C_6$ sulfoalkylether β-cyclodextrin and mixtures thereof.

In a most preferred embodiment the cyclodextrin is a β-cyclodextrin selected from the group consisting of $C_1$-$C_6$ hydroxyalkyl-β-cyclodextrins, $C_2$-$C_6$ sulfoalkylether β-cyclodextrins and mixtures thereof.

In the examples of the present invention the following β-cyclodextrins are used:

- β-CD: β-cyclodextrin (Sigma-Aldrich Ref.: C4767-25G).
- HP-β-CD: 2-hydroxypropyl-β-cyclodextrin with a degree of substitution of 5.6 (Sigma-Aldrich Ref.: 332607-5G).
- HPB: 2-hydroxypropyl-β-cyclodextrin with a degree of substitution of 4.5 (Kleptose™ Roquette Pharma).
- CM-β-CD: Carboxymethyl-β-cyclodextrin sodium salt with a degree of substitution of 3 (Sigma-Aldrich Ref.: 21906-5G).
- DM-β-CD: Heptakis(2,6-di-O-methyl)-β-cyclodextrin (Sigma-Aldrich Ref.: H0513-5G).
- SBE-β-CD: β-Cyclodextrin sulfobutylether sodium salt - USP with a degree of substitution of 6.2 - 6.9 (Carbosynth Ref.: SBECD).

In a preferred embodiment the cyclodextrin is a pharmaceutically acceptable cyclodextrin.

In a particular embodiment, the cyclodextrin is present in the composition in an amount from about 0.1% to about 50% w/v. Here, w/v means weight/volume percentage concentration (g/100 mL), e.g. when the cyclodextrin is present in the composition in an amount from about 0.1% to about 50% means that is present in an amount from about 1 mg/mL to about 500 mg/mL. In the present invention, all the percentages are in w/v units unless otherwise stated. In specific embodiments, the cyclodextrin is present in an amount from about 0.25% to about 30%, from about 0.5% to about 25%, from about 1% to about 20%, from about 2% to about 15%, or from about 3% to about 10% w/v. In a preferred embodiment, the cyclodextrin of the pharmaceutical composition of the invention is in a concentration of at least 5% w/v but no more than 15% w/v, i.e. from about 50 to 150 mg/mL. More preferably, the cyclodextrin is present in an amount of at least 8% but no more than 10% w/v. In another preferred embodiment, the composition of the invention comprises 9 % w/v of cyclodextrin.

*Gelling agent*

Water-soluble gelling agents refer to substances which can increase the viscosity of an aqueous solution such as the ophthalmic pharmaceutical composition of the invention, without substantially changing its other properties, but which form a gel, dissolving in the liquid phase as a colloid mixture that forms a weakly cohesive internal structure. In a preferred embodiment the at least one gelling agent is a pharmaceutically acceptable gelling agent for ophthalmic purposes.

In the present invention, the at least one pharmaceutically acceptable water-soluble gelling agent or an acceptable salt thereof is selected from hyaluronic acid, Gellan gum, and mixtures thereof.

Therefore, in a preferred embodiment, the at least one pharmaceutically acceptable water-soluble gelling agent is hyaluronic acid or an acceptable salt thereof.

In a particular embodiment the gelling agent or an acceptable salt thereof is present in the aqueous composition of the invention in an amount from about 0.001% w/v to about 2% w/v, preferably from about 0.003% w/v to about 1% w/v. In particular embodiments the gelling agent is present in the aqueous composition of the invention in an amount of about 0.05%, about 0.1%, about 0.25 %, about 0.50%, about 0.75%, about 1%, about 1.5%, or about 2%. All the percentages are in w/v units unless otherwise stated. In a preferred embodiment, the gelling agent is present in the aqueous composition of the invention in an amount of at least 0.05% w/v but no greater than 1% w/v, most preferably at 0.1 % w/v.

In a preferred embodiment, the ophthalmic pharmaceutical composition of the invention comprises:

a) at least 0.6% w/v but no more than 1.0% w/v of bilastine, or a pharmaceutically acceptable salt or solvate thereof, wherein the bilastine or said salt or solvate thereof is completely dissolved in the aqueous ophthalmic pharmaceutical composition;

b) at least one β-cyclodextrin, selected from the group consisting of unmodified β-cyclodextrin, $C_1$-$C_6$ alkyl-β-cyclodextrin, $C_1$-$C_6$ hydroxyalkyl β-cyclodextrin, $C_1$-$C_6$ carboxyalkyl-β-cyclodextrin, carbonyl-β-cyclodextrin, $C_2$-$C_6$ sulfoalkylether β-cyclodextrin and mixtures thereof, wherein the concentration of said β-cyclodextrin is at least 5% w/v but no more than 15% w/v; and

c) hyaluronic acid or a pharmaceutically acceptable salt thereof, wherein the concentration of the hyaluronic acid or a pharmaceutically acceptable salt thereof is at least 0.05% w/v but no more than 1% w/v.

In a particular embodiment, the gelling agent is hyaluronic acid or a pharmaceutically acceptable salt thereof and has a molecular weight no greater than 600000 Da. The molecular weight of the gelling agent can be measured according to known techniques in the art. Preferably but not limited, the average molecular weight of the hyaluronic acid or a pharmaceutically acceptable salt thereof may be determined using size exclusion chromatography coupled to multiangle laser light scattering (SEC-MALLS). Alternatively, the average molecular weight of the hyaluronic acid or a pharmaceutically acceptable salt thereof may also be determined using the intrinsic viscosity and the Mark-Houwink relation.

Further embodiments

In a particular embodiment, the ophthalmic pharmaceutical composition of the invention further comprises at least one pharmaceutically acceptable water-soluble polymer as a viscosity agent, selected from the group consisting of an ether derivative of cellulose, polyethylene glycol, polyvinyl alcohol, and mixtures thereof.

Water-soluble polymers refer to hydrophilic polymers which are, at least partially, soluble in water. In a preferred

embodiment the at least one water-soluble polymer is a pharmaceutically acceptable water-soluble polymer.

In a preferred embodiment the at least one water-soluble-polymer is an ether derivative of cellulose, most preferably methylcellulose. In another embodiment the at least one water-soluble polymer is polyethylenglycol.

In a particular embodiment the water-soluble polymer is present in the aqueous composition of the invention in an amount from about 0.001% w/v to about 15% w/v, preferably from about 0.01% w/v to about 15% w/v. In particular embodiments the water-soluble polymer is present in the aqueous composition of the invention in an amount about 0.01%, about 0.05%, about 0.1%, about 0.25 %, about 0.50%, about 0.75%, about 1%, about 3%, about 5%, about 7%, about 10%, about 13% or about 15%. All the percentages are in w/v units unless otherwise stated.

- Cellulose ether derivatives

In one embodiment the water-soluble polymer is an ether derivative of cellulose. A cellulose ether derivative refers to cellulose wherein the hydroxyl groups of cellulose have been partially or fully substituted to provide cellulose ethers (-OR). In one embodiment the ether derivative of cellulose is selected from the group consisting of alkyl celluloses, hydroxyalkyl celluloses, carboxyalkyl celluloses and mixtures thereof.

In a preferred embodiment, the ether derivative of cellulose is selected from the group consisting of hydroxypropylcellulose, hydroxypropylmethylcellulose, hydroxyethylcellulose, methylcellulose, carboxyalkyl cellulose and mixtures thereof.

In one embodiment the cellulose ether derivative is alkyl cellulose. Preferred alkyl celluloses for use herein include methylcellulose (MC), ethylcellulose, ethylmethyl cellulose and mixtures thereof.

In one embodiment the cellulose ether derivative is a hydroxyalkyl cellulose. Preferred hydroxyalkyl celluloses for use herein include hydroxymethyl cellulose (HMC), hydroxyethyl cellulose (HEC), hydroxypropyl cellulose (HPC), hydroxypropylmethyl cellulose (HPMC) and ethyl hydroxyethyl cellulose.

In one embodiment the cellulose ether derivative is a carboxyalkyl cellulose. Preferred carboxyalkyl celluloses for use herein include carboxymethyl cellulose (CMC). CMC and sodium carboxymethyl cellulose (CMCNa) are equivalents.

- Polyethylene glycol (PEG)

PEG is also known as polyethylene oxide (PEO) or polyoxyethylene (POE). In one preferred embodiment PEG is a low-molecular-weight PEG, which means PEG having molecular-weight between 300-1000 g/mol. The molecular weight of PEG is more preferably between 300 and 500 g/mol. In a preferred embodiment the molecular-weight is 400 g/mol, i.e., PEG400.

In a particular embodiment, the composition has an osmolality comprised between about 200 mOsm/kg to about 640 mOsm/kg, preferably about 250 mOsm/kg to about 600 mOsm/kg. In a preferred embodiment, the osmolality is about 240 mOsm/kg to about 340 mOsm/kg. The osmolality of the ophthalmic pharmaceutical solution of the invention may be measured using standard methods well-known in the art. Preferably but not limited, the osmolality of the ophthalmic pharmaceutical solution of the invention may be determined by the measurement of the freezing point depression of the solution with an osmometer.

In a particular embodiment, the composition of the invention can further comprise osmolality agents or tonicity agents selected from glycerin, sorbitol, mannitol, erythriol, arabitol, xylitol, ribitol, galactitol, multitol, macrogol, lactitol, and mixtures thereof. When present, said osmolality agents or tonicity agents are in the aqueous composition of the invention in an amount of about 0.05%, about 0.1%, about 0.25 %, about 0.50%, about 0.75%, about 1%, about 1.5%, about 1.6%, about 2%, about 3%, about 5%, about 7%, about 10%, about 13% or about 15%. In an embodiment compatible with the former, said osmolality agents or tonicity agents are in the aqueous composition of the invention in an amount of less than 15%, 13%, 10%, 7%, 5%, 3% or 2%. All the percentages are in w/v units unless otherwise stated.

In a preferred embodiment, when present, the osmolality agents or tonicity agents are in the aqueous composition of the invention in an amount of between 0.05% and 5% w/v.

In a particular embodiment, the inventors have surprisingly found that the composition of the invention is stable and advantageously does not require the addition of preservative agents, such as benzalkonium chloride, imidazolidinyl urea, methylparaben, propylparaben, phenoxyethanol, disodium EDTA, thimerosal, chlorobutanol and sorbic acid, which are known to cause dry eye and eye irritation. Thus, in a particular embodiment, the ophthalmic pharmaceutical composition of the invention is devoid of preservatives.

In a particular embodiment, the composition of the invention comprises glycerin. Glycerin is synonym to glycerol or glycerine. Preferably, the glycerin is in the aqueous composition of the invention in an amount between 0.05% and 5% w/v, more preferably between 0.05% and 3% w/v. In a preferred embodiment, the glycerin is present as a tonicity agent at a concentration not greater than 2.5%. In another preferred embodiment, the composition of the invention comprises glycerin in an amount of about 1.6%, preferably of 1.61%. All the percentages are in w/v units unless otherwise

stated.

*pH*

The aqueous pharmaceutical composition of the invention has been preferably developed for ophthalmic uses and/or administration, i.e. the aqueous ophthalmic pharmaceutical composition is adapted to these purposes. The physiological pH of the eye, particularly the human eye, is known to be between about 6.5-8.0.

In a preferred embodiment, the pharmaceutical composition has a pH value comprised between 4 and 9, both lower and upper limits included. In some embodiments the pH of the aqueous ophthalmic pharmaceutical composition of the present invention is 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, 9.0, 9.1, 9.2, 9.3 or 9.4.

In the context of pH values, it must be readily understood that a pH value of 4 may also identified as corresponding to a pH range of from 3.6 to 4.4. Similarly, a pH value of 9 may also be identified as corresponding to a pH range of from 8.6 to 9.4. In another preferred embodiment, the pH value of the pharmaceutical composition is 4.0, 5.5, 7.4, 8.0 or 9.0.

In one embodiment there is present a pH-adjusting agent selected from the group consisting of hydrochloric acid, boric acid, acetic acid, sodium hydroxide, potassium hydroxide, or a combination thereof.

In one embodiment there is present a buffering agent selected from the group consisting of acetate buffer, a citrate buffer, a phosphate buffer, a borate buffer, or a combination thereof.

In another embodiment the composition has a pH in a range that maintains chemical, physical, and/or physiological stability of bilastine and is well-tolerated by the eye.

*Uses*

Bilastine has been found to be an antagonist of histamine $H_1$ receptor and would thus be useful in the treatment and/or prevention of diseases known to be susceptible to amelioration by antagonism of histamine $H_1$ receptor.

Therefore, an aspect of the invention refers to an aqueous ophthalmic pharmaceutical composition as defined above for use in the treatment and/or prevention of a disorder or disease susceptible to amelioration by antagonism of $H_1$ histamine receptor, selected from an ocular allergic disorder or an allergic disease..

In a preferred embodiment, the invention is directed to an aqueous ophthalmic pharmaceutical composition as defined above for use in the treatment and/or prevention of an ocular allergic disorder or allergic disease. Preferably, an allergic disease or disorder selected from rhinitis, rhinoconjunctivitis, allergic conjunctivitis, vernal keratoconjunctivitis, atopic keratoconjunctivitis, giant papillary conjunctivitis, ocular irritation, itchiness, redness, tearing, chemosis, keratitis sicca, keratoconjunctivitis sicca or dysfunctional tear syndrome. In the context of the present invention, the dry-eye syndrome includes keratitis sicca, keratoconjunctivitis sicca or dysfunctional tear syndrome.

Preferably, the invention is directed to an aqueous ophthalmic pharmaceutical composition as defined above for use in the treatment and/or prevention of allergic conjunctivitis. In another preferred embodiment, the invention is directed to an aqueous ophthalmic pharmaceutical composition as defined above for use in the treatment and/or prevention of dry eye syndrome. In a more preferred embodiment, the invention is directed to an aqueous ophthalmic pharmaceutical composition as defined above for use in the simultaneous treatment and/or prevention of allergic conjunctivitis and dry eye syndrome.

The term "treatment" or "to treat" in the context of this specification means administration of a compound or formulation according to the invention to ameliorate or eliminate the disease or one or more symptoms associated with said disease. "Treatment" also encompasses ameliorating or eliminating the physiological sequelae of the disease.

The term "ameliorate" in the context of this invention is understood as meaning any improvement on the situation of the patient treated.

The term "prevention" or "to prevent" in the context of this specification means administration of a compound or formulation according to the invention to reduce the risk of acquiring or developing the disease or one or more symptoms associated with said disease.

*Pharmaceutical composition*

The expression "aqueous ophthalmic pharmaceutical composition" refers to a liquid pharmaceutical composition comprising water suitable for ocular use.

In one embodiment, bilastine is completely dissolved in the aqueous ophthalmic pharmaceutical composition of the invention at a concentration of at least 0.4% w/v. In another embodiment, bilastine is completely dissolved in the aqueous ophthalmic pharmaceutical composition of the invention at a concentration of at least 0.6% w/v, preferably at least 0.7%. In another embodiment, bilastine is completely dissolved in the aqueous ophthalmic pharmaceutical composition of the invention at a concentration comprised between 0.6 to 1.0 w/v %, preferably at a concentration comprised between 0.6 to 0.9 w/v %, more preferably at a concentration comprised between 0.6 to 0.8%. Preferably, bilastine is completely dissolved in the aqueous ophthalmic pharmaceutical composition of the invention at a concentration of 0.6 % w/v. Here, w/v % means weight/volume percentage concentration (g/100 mL), e.g. when bilastine is present in the composition in an amount from about 0.6% to about 1.0% means that is present in an amount from about 6 mg/mL to about 10 mg/mL. In the present invention, all the percentages are in w/v units unless otherwise stated.

In one embodiment, the amount of bilastine in the pharmaceutical composition of the invention is preferably above 4500 μg/mL, preferably above 6000 μg/mL, preferably above 6500 μg/mL, preferably above 7000 μg/mL, preferably above 7500 μg/mL, preferably above 8000 μg/mL, preferably above 8500 μg/mL, preferably above 9000 μg/mL, and more preferably above 9500 μg/mL. In a preferred embodiment, the amount of bilastine in the pharmaceutical composition of the invention is lower than 10500 μg/mL

In a particular embodiment the aqueous ophthalmic pharmaceutical composition comprises bilastine, a β-cyclodextrin and hyaluronic acid or an acceptable salt thereof, wherein the bilastine is completely dissolved in the aqueous ophthalmic pharmaceutical composition. In another particular embodiment the aqueous ophthalmic pharmaceutical composition comprises bilastine, hydroxypropyl-β-cyclodextrin and hyaluronic acid or an acceptable salt thereof, wherein the bilastine is completely dissolved in the aqueous ophthalmic pharmaceutical composition.

The expression "therapeutically effective amount" means that amount of a medicament which when administered supplies an amount of one or more pharmaceutically active agents contained therein to provide a therapeutic benefit in the treatment or management of a disease or disease state.

In a preferred embodiment the pharmaceutical composition of the invention comprises a therapeutically effective amount of bilastine.

The aqueous ophthalmic pharmaceutical composition of the invention may comprise further pharmaceutically acceptable excipients.

The expression "pharmaceutically acceptable excipient" refers to a vehicle, diluent, or adjuvant that is administered with the active ingredient. Such pharmaceutical excipients can be sterile liquids, such as water and oils, including those of petroleum, animal, vegetable, or synthetic origin, such as peanut oil, soybean oil, mineral oil, sesame oil, and similar. Water or saline aqueous solutions and aqueous dextrose and glycerin solutions, particularly for injectable solutions, are preferably used as vehicles. Suitable pharmaceutical vehicles are described in "Remington's Pharmaceutical Sciences" by E.W. Martin, 21st Edition, 2005.

The excipients and auxiliary substances necessary to manufacture the desired pharmaceutical form of administration of the pharmaceutical composition of the invention will depend, among other factors, on the elected administration pharmaceutical form. Said pharmaceutical forms of administration of the pharmaceutical composition will be manufactured according to conventional methods known by the skilled person in the art. A review of different active ingredient administration methods, excipients to be used and processes for producing them can be found in "Tratado de Farmacia Galénica", C. Faulí i Trillo, Luzán 5, S.A. de Ediciones, 1993.

The expression "pharmaceutically acceptable" refers to compositions and molecular entities that are physiologically tolerable and do not typically produce an allergic reaction or a similar unfavourable reaction as gastric disorders, dizziness and suchlike, when administered to a human or animal. Preferably, the term "pharmaceutically acceptable" means it is approved by a regulatory agency of a state or federal government or is included in the U.S. Pharmacopoeia or other generally recognized pharmacopoeia for use in animals, and more particularly in humans.

Formulations disclosed herein also optionally further comprise one or more ophthalmic excipients. Ophthalmic excipients include, by way of non-limiting examples, at least one agent selected from a mucoadhesive, a preservative, a pH-adjusting agent, a tonicity-adjusting agent, a buffering agent, an antioxidant, a chelating agent, an antimicrobial preservative, a chemical preservative, a viscosity agent or a combination thereof. In a particular embodiment, the ophthalmic composition further comprises a water-soluble polymer, as defined above.

In a particular embodiment the composition of the invention does not comprise a corticosteroid.

In a preferred embodiment, the ophthalmic pharmaceutical composition comprises:

a) at least 0.6% w/v but no more than 1.0% w/v of bilastine or a pharmaceutically acceptable salt or solvate thereof, wherein the bilastine or said salt or solvate thereof is completely dissolved in the aqueous ophthalmic pharmaceutical composition;

b) at least one $C_1$-$C_6$ hydroxyalkyl β-cyclodextrin, wherein the concentration of said β-cyclodextrin is at least 5% w/v but no more than 15% w/v;

c) hyaluronic acid or a pharmaceutically acceptable salt thereof, wherein the concentration of said hyaluronic acid or a pharmaceutically acceptable salt thereof is at least 0.05% w/v but no more than 1% w/v;

d) from 0.001% w/v to 15% w/v of at least one pharmaceutically acceptable water-soluble polymer, selected from the group consisting of an ether derivative of cellulose, polyethylene glycol, polyvinyl alcohol, and mixtures thereof; and

e) from 0.05% w/v to 5% w/v of at least one tonicity agent selected from the group consisting of glycerin, sorbitol, mannitol, erythriol, arabitol, xylitol, ribitol, galactitol, multitol, macrogol, lactitol, and mixtures thereof.

In another preferred embodiment, the ophthalmic pharmaceutical composition comprises:

a) at least 0.6% w/v but no more than 1.0% w/v of bilastine or a pharmaceutically acceptable salt or solvate thereof, wherein the bilastine or said salt or solvate thereof is completely dissolved in the aqueous ophthalmic pharmaceutical composition;

b) at least one $C_1$-$C_6$ hydroxyalkyl β-cyclodextrin, wherein the concentration of said β-cyclodextrin is at least 5% w/v but no more than 15% w/v;

c) hyaluronic acid or a pharmaceutically acceptable salt thereof, wherein the concentration of said hyaluronic acid or a pharmaceutically acceptable salt thereof is at least 0.05% w/v but no more than 1% w/v;

d) from 0.001% w/v to 15% w/v of an ether derivative of cellulose; and

e) from 0.05% w/v to 5% w/v of glycerin.

In a more preferred embodiment, the ophthalmic pharmaceutical composition comprises:

a) at least 0.6% w/v but no more than 1.0% w/v of bilastine or a pharmaceutically acceptable salt or solvate thereof, wherein the bilastine or said salt or solvate thereof is completely dissolved in the aqueous ophthalmic pharmaceutical composition;

b) at least one $C_1$-$C_6$ hydroxyalkyl β-cyclodextrin, wherein the concentration of said β-cyclodextrin is at least 5% w/v but no more than 15% w/v;

c) hyaluronic acid or a pharmaceutically acceptable salt thereof, wherein the concentration of said hyaluronic acid or a pharmaceutically acceptable salt thereof is at least 0.05% w/v but no more than 1% w/v;

d) from 0.005% w/v to 0.1% w/v of methylcellulose; and

e) from 0.5% w/v to 2% w/v of glycerin.

Table 1 below provides a listing of exemplary ingredients suitable for an exemplary preferred formulation of the ophthalmic composition of the present invention and a desired weight/volume percentage for those ingredients. It shall be understood that the following Table 1 is exemplary and that certain ingredients may be added or removed from the Table and concentrations of certain ingredients may be changed while the formulation can remain within the scope of the present invention, unless otherwise specifically stated.

Table 1. Exemplary preferred formulation of the ophthalmic composition of the present invention and a desired weight/volume percentage for those ingredients.

| Ingredient | w/v % |
|---|---|
| Bilastine | 0.6 |
| β-Cyclodextrin (Hydroxypropyl-β-Cyclodextrin) | 9.0 |
| Gelling agent (Sodium hyaluronate) | 0.1 |
| Tonicity agent (Glycerin) | 1.61 |
| Viscosity agent (Methyl cellulose) | 0.01 |
| pH adjusting agent (NaOH or HCl) | sufficient to achieve pH = 7.4 |

(continued)

| Ingredient | w/v % |
|---|---|
| purified water | Q.S. 100 |

*Pharmaceutical forms*

Examples of pharmaceutical compositions include any liquid composition for topical administration to the eye. Liquid forms are solutions, suspensions or emulsions.

Examples of suitable preparations for topical administration to the eye include ophthalmic drops (i.e. eye drops or artificial tears), ophthalmic emulsions, and ophthalmic ointments. In a particular embodiment the compositions of the present invention are in form of ophthalmic preparations as ophthalmic drops. Ophthalmic preparations may include a suitable antimicrobial agent. In a preferred embodiment the ophthalmic preparations do not include a preservative. In a more preferred embodiment, the ophthalmic preparations do not include a preservative selected from benzalkonium chloride, imidazolidinyl urea, methylparaben, propylparaben, phenoxyethanol, disodium EDTA, thimerosal, chlorobutanol and sorbic acid.

The present invention provides an ophthalmic pharmaceutical composition as defined above. In a preferred embodiment, said ophthalmic pharmaceutical composition is a once-daily pharmaceutical composition.

The following examples are merely illustrative of certain embodiments of the invention and cannot be considered as restricting it in any way.

## EXAMPLES

### Materials and methods

The following materials have been used: bilastine (supplied by Sai Life Sciences, Batch 5000011325); the cyclodextrins described above as β-CD, HP-β-CD, HPB, CM-β-CD, DM-β-CD and SBE-β-CD; methylcellulose 1500 (MC1500) (Acofarma); Gellan gum (GG) (Kelcogel CG-LA, CPKelco), sodium hyaluronate (Caref) and glycerin (Merck-Millipore). The water used in the following examples was purified water obtained using an Elix® water purification system from Merck-Millipore.

### Example 1. Stability of over 6.5 mg/mL bilastine at 25°C, in the presence of 9% of different β-cyclodextrins in aqueous solutions at different pH values.

This example shows the stability of bilastine solutions at a concentration of over 6.5 mg/mL (0.65 % w/v) in the presence of 9.0% w/v of the five cyclodextrins β-CD, HP-β-CD, CM-β-CD, DM-β-CD and SBE-β-CD in aqueous solutions at different pH values. Each pH value was obtained by adjustment starting from a basic solution as follows.

Bilastine in non-buffered solutions (pH adjustment).

a. 7 mg/mL solution of bilastine in basic media: 70 mg of bilastine were added to a 10 mL volumetric flask. Approximately 5 mL of deionised water were added and then NaOH 1M was added dropwise while shaking the mixture until all the bilastine was dissolved. Then, 900 mg of the corresponding cyclodextrin were added and the mixture was shaken until complete solubilisation of the cyclodextrin (except in the specific case of β-CD which did not lead to full solubilisation). The volume was then adjusted to 10 mL.

b. 900 μL of the five bilastine+cyclodextrin solutions prepared in step a. were each transferred into separate Eppendorf tubes.

c. In each Eppendorf, the pH was adjusted to the desired value by careful addition of AcOH 50%, HCl 1M or NaOH 1M as required.

to = 0 days

The solutions were kept under stirring at 30 °C for 1 hour after which the concentration bilastine was measured.

$t_1$ = 7 days

After the analysis at $t_0$, the solutions were kept for a period of 7 days in a thermostated room at 22 ± 2°C without stirring and reanalysed.

Each Eppendorf was introduced into an incubator (VWR® Incubating Mini Shaker) under constant stirring (100 rpm) and it was incubated at 30 °C for 1 hour. Then, 1.5 mL samples of each Eppendorf were centrifuged for 0.5 hours at 12,500 rpm (SIGMA 2-16P thermostated centrifuge) to eliminate solid particles of bilastine. Samples of the supernatant were appropriately diluted (1:100) to determine the concentration of bilastine. The concentration of bilastine was measured using a diode array spectrophotometer Hewlett Packard 8452A. Assays were performed in triplicate. Results are shown below.

Table 2. Concentration of bilastine (target 7 mg/mL) in the presence of 9% w/v of different cyclodextrins in non-buffered aqueous solutions at different pH values at to.

| pH | 4.0 | 5.5 | 7.4 | 8.0 | 9.0 |
|---|---|---|---|---|---|
| β-CD | 7.0 | 7.0 | 6.9 | 7.0 | 6.9 |
| HP-β-CD | 6.4 | 6.6 | 6.6 | 6.6 | 6.7 |
| CM-β-CD | 6.5 | 6.6 | 6.7 | 6.7 | 6.8 |
| DM-β-CD | 6.7 | 6.8 | 6.8 | 6.7 | 6.8 |
| SBE-β-CD | 6.6 | 6.8 | 6.9 | 6.7 | 6.8 |

The deviation from 7 mg/mL in the concentration values of bilastine is due to the dilution effect after the pH adjustment step.

Table 3. Concentration of Bilastine (target 7 mg/mL) in the presence of 9% w/v of different cyclodextrins in non-buffered aqueous solutions at different pH values at $t_1$ (7 days).

| pH | 4 | 5.5 | 7.4 | 8 | 9 |
|---|---|---|---|---|---|
| β-CD | 7.2 | 7.3 | 7.2 | 7.1 | 7.2 |
| HP-β-CD | 6.7 | 6.8 | 6.8 | 6.9 | 6.8 |
| CM-β-CD | 6.6 | 6.8 | 6.8 | 6.8 | 6.9 |
| DM-β-CD | 6.7 | 6.9 | 6.8 | 6.9 | 6.9 |
| SBE-β-CD | 6.6 | 6.8 | 6.9 | 6.8 | 6.8 |

After 1 week the values are still over 6,5 mg/mL. In comparison with the results at to, there is a small but general increase in the concentration of Bilastine which is caused by the slight evaporation of solvent.

This Example shows that a bilastine concentration of over 6.5 mg/mL is stable in non-buffered solutions after 1 hour at 30 °C and after 7 days at 22 °C for the pH range of 4.0 to 9.0. These results are represented in Figure 1 (to) and Figure 2 ($t_1$).

**Example 2. Ocular bioadhesion of bilastine 0.4% w/v and HPB 9% in varying compositions**

The bioadhesion of bilastine 0.4% w/v and HPB 9% compositions in the eye of animals (rat) was measured by positron emission tomography (PET) scans. The compositions varied in their content of viscosity and tonicity agents: methylcellulose and glycerin; and gelling agent: sodium hyaluronate and/or Gellan gum. The addition of the positron-emitting tracer $^{18}$FDG allowed measuring the average retention time of each composition in the rat's ocular tissue.

Table 4. Ocular Average Retention Time (ART) of compositions of the invention comprising bilastine 0.4% w/v and HPB 9%.

| Glycerin | Methylcellulose | Gellan gum | Sodium hyaluronate | ART/min |
|---|---|---|---|---|
|  | --- | --- | --- | 80.8 |
| 1.61% | 0.01% | --- | --- | 59.9 |
|  | --- | 0.2% | --- | 99.7 |
| 1.61% | 0.01% | 0.2% | --- | 108.7 |
|  | --- | --- | 0.1% | 110.4 |

(continued)

| Glycerin | Methylcellulose | Gellan gum | Sodium hyaluronate | ART/min |
|---|---|---|---|---|
| 1.61% | 0.01% | --- | 0.1% | 105.7 |
| | --- | 0.2% | 0.1% | 111.3 |
| 1.61% | 0.01% | 0.2% | 0.1% | 107.2 |

Table 4 shows that the longest retention time, or ocular bioadhesion, is achieved when sodium hyaluronate (HA), Gellan gum (GG) or a mixture of AH and GG are used in combination with bilastine and HPB cyclodextrin both with and without methylcellulose (MC) and glycerin. Therefore, the results show that at least GG or HA are suitable for increasing the ocular retention time of the composition comprising bilastine and HPB and, optionally, MC and glycerin. The results are shown in Figure 3.

Example 3. Preclinical ***in vivo* efficacy of ophthalmic formulations**

**ANIMAL MODEL**

Male Dunkin-Hartley guinea pigs, weighing 300-349 g at reception (Harlan Laboratories Inc./Envigo) and kept under standard housing conditions (2 animals/cage) were used. Animals (6-10 per group) were reused after a *washing* period of at least 4 days between two consecutive uses to enable the complete recovering of the ocular conjunctiva.

*Experimental procedure*

Two aqueous ophthalmic formulations of bilastine were prepared at a bilastine concentration of 0.4% and 0.6% w/v. Both formulations also comprised HPB 90 mg/mL, methylcellulose 0.1 mg/mL, sodium hyaluronate 1 mg/mL and glycerin 16.1 mg/mL.

The ophthalmic formulations were applied by instillation in the right eye (25 μL), maintaining the animal immobilized for 2 to 3 minutes to ensure the permanence of the product in the conjunctival sac. Next, the left eye received the same volume of the correspondent vehicle or NaCl 0.9% in the case of marketed eye drops. Each animal was its own positive control.

Once the established time had elapsed (it could be from 10 min to 24 h), acute conjunctivitis was induced by the administration in each eye of a histamine dihydrochloride solution (5% in NaCl 0.9%, 25 μL/eye), maintaining the animal immobilized for 2 to 3 minutes. The response to histamine was evaluated 30 min after its application.

The following parameters were scored for each eye: the degree of oedema (conjunctival chemosis), the conjunctiva reddeness and vascular injection (conjunctival hyperemia) and the lacrimal secretion (epiphora). The degree of severity was evaluated according to a subjective scale from 0 to 4 (Guideline OECD Test N° 405: 0 = normal; 1 = minimum; 2 = moderate; 3 = intense; 4 = severe). The result of adding the individual scores of each parameter in each eye (total score between 0 and 12) was considered as the average of the global conjunctivitis degree.

*Statistics*

Formulations that did not reach statistical significance or those reaching statistical significance but that did antagonize the histamine effect in a percentage ≤ 30% were considered inactive. For each treatment, the mean values of total score in the control eye (left) and in the antihistamine-treated eye (right) were calculated. In addition, the percentage of inhibition (average of the pharmacological effect) was calculated according to the next formula:

$$\text{Inhibition (\%)} = [(\text{control score} - \text{treatment score}) / \text{control score}] \times 100$$

Statistical analysis involved the use of the non-parametric tests "Wilcoxon Signed Rank test" (paired data) and the "Mann-Whitney U-Test" (for independent data), in order to compare each treatment with its correspondent control and differences between treatments, respectively. Statistically significant differences were considered when p<0.05.

**Effect of commercial formulations**

Azelastine 0.5 mg/ml (Afluon®) was active 9 h after application (38.4 % inhibition of histamine-induced effects), but not after 12 h (19.2% inhibition). Olopatadine 1 mg/mL (Opatanol®) provided a much longer duration of activity, as

its inhibitory activity was observed 24 hours after dosing (38.0% inhibition). The observed differences between azelastine and olopatadine in this model (see Figure 4), agree with previous evidence comparing the duration of antihistaminic activity of marketed drugs for the topical treatment of seasonal allergic conjunctivitis, using a guinea pig model of histamine-induced conjunctival vascular permeability (Beauregard C, Stephens D, Roberts L, Gamache D and Yanni J. Duration of action of topical antiallergy drugs in a Guinea pig model of histamine-induced conjunctival vascular permeability. J Ocul Pharmacol Ther. 2007, 23: 315-20). Interestingly, the extended duration of action of an olopatadine 0.2% solution in this histamine-induced vascular leakage animal model was confirmed in a human clinical trial (Vogelson CT, Abelson MB, Pasquine T, Stephens DM, Gamache DA, Gross RD, Robertson SM, Yanni JM. Preclinical and clinical antiallergic effect of olopatadine 0.2% solution 24 hours after topical ocular administration. Allergy Asthma Proc. 2004, 25: 69-75).

**Effect of concentrations of bilastine**

A formulation of bilastine 4 mg/mL (0.4% w/v) described above was prepared and the duration of action was determined. In a similar manner to azelastine 0.5 mg/mL, the activity obtained with this formulation against histamine-induced conjunctivitis in guinea pigs remains significant up to 9 hours after its application (36.4% inhibition, Figure 4).

A formulation of bilastine 6 mg/mL (0.6% w/v) described above was also prepared and its activity compared with that of the bilastine 4 mg/mL. The results obtained indicate a scarce improvement in terms of inhibition percentages with the highest bilastine concentration both after 9 hours (40.5 and 36.4% inhibition) and 12 h dosing (29.0 and 20.4% inhibition). No statistical differences were found at any time. At the same time intervals, olopatadine 1 mg/mL showed a strong activity (75.9 and 63.9% of inhibition at 9 and 12 hours after dosing respectively). It was concluded that the increase in the concentration of bilastine did not entail a notable improvement in terms of effectiveness and duration of the effect.

**Effect of methylcellulose**

The effectiveness of equivalent bilastine 0.4% w/v formulations with and without methylcellulose 0.1 mg/mL was compared. Both formulations also comprised HPB 100 mg/mL, sodium hyaluronate 1 mg/mL and Gellan gum 2 mg/mL. At 9 hours after dosing both formulations resulted clearly active (44.7 vs 47.4% of antagonism of histamine-induced effects respectively). These results indicate that methylcellulose does not contribute in a relevant manner to the duration of the activity of the bilastine 0.4% w/v formulation.

**Effect of glycerin**

The effectiveness of equivalent bilastine 0.4% w/v formulations with and without glycerin 16.1 mg/mL was compared. Both formulations also comprised HPB 100 mg/mL, methylcellulose 0.1 mg/mL, sodium hyaluronate 1 mg/mL and Gellan gum 2 mg/mL. At 9 hours after dosing both formulations resulted active (33.5 vs 45.8% of inhibition for formulations with and without glycerin respectively). At 12 hours dosing both formulations resulted clearly inactive (24.5 vs 26.7% of antagonism respectively). No statistical differences were found at any time. These results indicate that glycerin does not contribute in a relevant manner to the duration of the activity of the bilastine 0.4% w/v formulation.

Example 4. Clinical ***in vivo*** **efficacy of ophthalmic formulations**

This Example, along with Tables 5-6 and Figures 5-6 show the results of a single-center, double-masked, randomized, vehicle-controlled, phase 2, dose ranging evaluation of the effectiveness of bilastine ophthalmic solution (0.2% w/v, 0.4% w/v, and 0.6% w/v) compared to vehicle for the treatment of allergic conjunctivitis in the Conjunctival Allergen Challenge (ORA-CAC®) model.

The aqueous ophthalmic formulations of bilastine were prepared at a bilastine concentration of 0.2% w/v, 0.4% and 0.6% w/v. The vehicle formulation did not comprise any bilastine. The excipients were as indicated above in Table 1, i.e., also comprised HPB 90 mg/mL, methylcellulose 0.1 mg/mL, sodium hyaluronate 1 mg/mL and glycerin 16.1 mg/mL.

The CAC® is a standardized model for studying investigational therapies for ocular allergy. The CAC® induces the signs and symptoms of ocular allergy (e.g. ocular redness, swelling tearing, and ocular itching) in a controlled manner by direct administration of allergen to the conjunctiva.

One dose (one drop in each eye) of the assigned test article was administered 15 minutes, 8, or 16 hours prior to a CAC® test and allergic conjunctivitis sign and symptom assessments. The study consisted of eight office visits over a period of approximately six to ten weeks. Efficacy was assessed using the CAC® model performed by ORA, Inc., Andover, Massachusetts, United States, 01810. The CAC® model includes a screening, treatment and follow-up period.

In the screening period, at Visit 1, subjects signed the informed consent and an allergic skin test was performed.

At Visit 2, each qualifying subject underwent a bilateral CAC® titration using an allergen they had a positive reaction to on their skin test. Subjects who elicited a positive reaction post-CAC® underwent the confirmation CAC® at Visit 3 using the same allergen they qualified with at Visit 2.

The treatment period began at Visit 4a after subjects were randomized. At this visit, subjects received an in-office dose of the treatment they were randomized to receive. Approximately 16 hours post-instillation of study medication, subjects underwent CAC® at Visit 4b. At Visit 5a, subjects received an in-office dose of the same study medication. Approximately 8 hours post-instillation of study medication, subjects underwent CAC® at Visit 5b. Subjects received a final dose of study medication at Visit 6 approximately 15 minutes prior to CAC®.

In the follow-up period, a telephone call follow-up was made on Day 29 (±3) to all subjects. Table 5 shows a summary of the Visit Schedule.

Table 5. Summary of the Visit schedule

| | |
|---|---|
| Visit 1 (Day -50 to Day -22): | Screening/ Informed Consent/ Skin Test |
| Visit 2 (Day -21 ± 3): | Titration CAC® |
| Visit 3 (Day -14 ± 3): | Confirmation CAC® |
| Visit 4a (Day 1): | Enrollment/Randomization/ In-Office Instillation |
| Visit 4b (Day 1; 16 hours from Visit 4a): | 16 Hour Duration of Action CAC® |
| Visit 5a (Day 15 ± 3): | In-Office Instillation |
| Visit 5b (Day 15 ± 3; 8 hours from Visit 5a): | 8 Hour Duration of Action CAC® |
| Visit 6 (Day 22 ± 3): | In-Office Instillation/ 15-Minute Onset of Action CAC® |
| Day 29 (± 3): | Follow-Up Telephone Call |

A total of 220 subjects were screened in order to enroll approximately 120 subjects at one (1) site. The primary objective was to measure ocular itching as main symptom of allergic conjunctivitis.

In the CAC® model, each patient is dosed with drug or vehicle and exposed to allergen at specific challenge times. The challenge times for the study were 15 minutes, 8 hours and 16 hours after dosing. Thereafter, itching was determined at determination times of 3, 5 and 7 minutes after challenge times at Visits 4b (16 hours post instillation of study medication), 5b (8 hours post instillation of study medication), and 6 (15 minutes post instillation). Patients were asked to rate their ocular itching on a scale of 0 to 4 (allowing half unit increments) to attain itching scores and in each score 0 was the least and 4 was greatest. Therefore, patients received three doses of drug or vehicle and each dose was followed by an allergen challenge and then ocular itching was evaluated by the subject as discussed.

Treatment success was defined as at least one concentration of bilastine ophthalmic solution showing clinical superiority over vehicle by at least 0.5 units for all 3 post-CAC® time points, and at least 1 unit for the majority of post-CAC® time points. The results of those determinations at those time points are provided in Table 6 and that data is provided as a graph in Figures 5 and 6.

Table 6. Mean ocular itching score treatment differences calculated as Active-Vehicle using least square means (LSMeans).

| Visit | Visit 4b (16-hour duration) | | | Visit 5b (8-hour duration) | | | Visit 6 (15-minute duration) | | |
|---|---|---|---|---|---|---|---|---|---|
| Time Point (minutes) | 3 | 5 | 7 | 3 | 5 | 7 | 3 | 5 | 7 |
| Bilastine 0.2% w/v | -0.776* | -0.861* | -0.742* | -1.097 | -1.118 | -1.205 | -1.859 | -1.657 | -1.463 |
| Bilastine 0.4% w/v | -0.857* | -0.926* | -0.843* | -1.340 | -1.206 | -1.152 | -1.812 | -1.459 | -1.283 |
| Bilastine 0.6% w/v | -1.444 | -1.649 | -1.546 | -1.694 | -1.685 | -1.762 | -2.137 | -1.984 | -1.831 |
| * No clinical significance in the treatment differences. | | | | | | | | | |

Bilastine 0.6% w/v had clinically significant treatment differences in the relief of ocular itching at 15 minutes (Visits 6), 8 hours (Visits 5b) and 16 hours (Visits 4b) post study medication instillation. Bilastine 0.2% w/v and 0.4% w/v had clinically significant treatment differences in the treatment of ocular itching at 15 minutes (Visits 6) and 8 hours (Visits 5b) post study medication instillation. All concentrations of bilastine showed statistically significant differences from vehicle in the treatment of ocular itching at all visits.

As can be seen from Table 6 and Figures 5-6, bilastine at a concentration of 0.6% w/v was the only tested concentration that was clinically and statistically efficacious when given 16 hours prior to CAC® (Visits 4b) for the treatment of ocular itching. This data is particularly surprising since, prior to this CAC® study, there was no indication that a formulation of bilastine at a concentration of 0.6% w/v would be highly effective reducing ocular itching at 16 hours post instillation.

In previous preclinical essays (such as the one in the previous Example), which evaluated the *in vivo* efficacy of bilastine ophthalmic formulations at a concentration of 4 mg/ml (0.4% w/v) and 6 mg/ml (0.6% w/v) in a guinea pig model of acute conjunctivitis, it was concluded that the increase in the concentration of bilastine from 0.4% to 0.6% w/v, did not entail a notable improvement in terms of effectiveness and duration of the effect. Bilastine 0.6% w/v was considered active 9 hours after application, but not after 12 hours. It was also confirmed that olopatadine 1 mg/mL (Opatanol®) provided a much longer duration of activity, as its inhibitory activity was observed 24 hours after dosing. Interestingly, the state of the art confirms in a human clinical trial the extended duration of action of an olopatadine 0.2% solution in this histamine-induced vascular leakage animal model (Vogelson CT, Abelson MB, Pasquine T, Stephens DM, Gamache DA, Gross RD, Robertson SM, Yanni JM. Preclinical and clinical antiallergic effect of olopatadine 0.2% solution 24 hours after topical ocular administration. Allergy Asthma Proc. 2004, 25: 69-75). In another clinical trial, the relief from ocular itching provided by an olopatadine 0.77% formulation is maintained throughout a period of 24 hours (Torkildsen G., Narvekar A., Bergmann M. Efficacy and safety of olopatadine hydrochloride 0.77% in patients with allergic conjunctivitis using a conjunctival allergen-challenge model. Clinical Ophthalmology 2015:9 1703-1713).

In another study, bilastine and olopatadine anti-histaminic effects were compared in a guinea pig model of histamine-induced conjunctival vascular permeability. To measure *in vivo* anti-histaminic activity, guinea pigs were given a subconjunctival histamine challenge after pre-treatment with topical drug or vehicle and i.v. loading with Evans blue dye. When bilastine was administered 2 to 8 hours before histamine challenge, 50% efficacy was not achieved, whereas olopatadine maintained an $ED_{50}$ below 0.1% (May 2008, Volume 49, Issue 13, ARVO Annual Meeting Abstract, Comparative Effects of Olopatadine, Bepotastine, and Bilastine on Conjunctival Mast Cell Stabilization and Histamine-Induced Vascular Permeability, C. Beauregard; D. J. Stephens; S. T. Miller; L. Roberts; D. A. Gamache; J. M. Yanni). Therefore, up to now, the state of the art clearly points that bilastine is not capable of equal the potency or duration of antihistamine action of olopatadine *in vivo*.

Surprisingly, however, the data in Table 6 and Figures 5-6 show that bilastine at a concentration of 0.6% w/v provides statistically significant relief of ocular itching at 16 hours relative to vehicle, as assessed by the CAC® model, supporting once-daily dosing of a formulation with a content of bilastine greater than 0.4% w/v in the treatment of ocular itching associated with allergic conjunctivitis.

Limitations of currently available medications, such as the need for multiple daily doses and ocular adverse effects, can lead to poor treatment adherence or treatment discontinuation, particularly if the negative impact on quality of life is viewed as a burden beyond that imposed by the allergic conjunctivitis itself. Furthermore, once-daily regimens have been shown to contribute significantly to patient compliance reducing the risk of missed doses and possibly improving treatment outcomes and symptom control.

Therefore, patients with difficulty managing their allergic conjunctivitis symptoms with one dose of their antiallergic ocular drops and consequently having to use a second dose may benefit from the increased convenience of a once-daily dosing regimen of a formulation with a content of bilastine of at least 0.4% w/v.

## <u>Example 5. Ocular ***in vivo* biodistribution of ophthalmic formulations**</u>

This Example confirms the results of example 4 by showing that the bilastine in the ophthalmic formulations of the invention, upon administration to rabbit eyes, is mainly found in the conjunctiva, when compared to the cornea, iris, retina and crystalline lens tissues.

The aqueous ophthalmic formulations of bilastine were prepared at a bilastine concentration of 0.6% w/v. The excipients were as indicated above in Table 1, i.e., also comprised HPB 90 mg/mL, methylcellulose 0.1 mg/mL, sodium hyaluronate 1 mg/mL and glycerin 16.1 mg/mL.

The results of this Example were obtained in compliance with the following Good Laboratory Practice standards:

- Real Decreto (Royal Decree) 1369/2000 of 19 July (Spain)
- OECD Principles of Good Laboratory Practice (as revised in 1997), ENV/MC/CHEM(98) 17;
- EC Commission Directive 2004/10/EC of 11 February 2004;
- Arrêté du 14 Mars 2000 (France), 2004/10/EC

- OECD ENV/JM/MONO (2002) 9, 25 June 2002
- FDA Guidance For Industry - Bioanalytical Method Validation - May 2018, and
- EMA Guideline on bioanalytical method validation EMEA/CHMP/EWP/192217/2009 Rev 1 Corr 2 - July 2011

42, 4-5 month-old Dutch Belted rabbits were selected as test subjects. The weight of the animals at treatment start was from 1.6 to 2.1 kg.

Animal care and husbandry:

| | |
|---|---|
| Acclimatization | 12-14 days |
| Veterinary examination | At arrival and before to start the treatment, the animals were examined by a veterinary surgeon. |
| Conditions | Optimum hygienic conditions behind a barrier system.<br>Air-conditioned with 14-16 air changes per hour, and continuously monitored environment with ranges for room temperature of 19-21°C and humidity between 50 and 85%. 12 hours fluorescent light/12 hours dark. |
| Accommodation | Individual Noryl cages (65.3 x 65.3 x 47 cm). |
| Diet | Pelleted standard Teklad 2030C rabbit diet ad libitum (supplied by Envigo RMS, S.L.). |
| Water | Tap water in bottles ad libitum |
| Environmental enrichment program | Different types of material specific for this species were supplied to reduce stress, enhance well-being and improve behavior. |

Animals received 30 μL of the ophthalmic formulation in each eye on a single occasion. The ophthalmic formulation was placed directly on both eyes of each animal by means of an automatic pipette. A new pipette tip was used for each eye.

The eyes of the test animals were not washed after instillation. Animals were weighed before administration and observed after administration to record any possible clinical sign.

Two to three animals were sacrificed at each time point (0.5, 1, 2, 4, 6, 8, 12 and 24 hours) and the following tissues from both eyes were collected: Aqueous humor, vitreous humor, cornea, conjunctiva, iris/ciliary body, crystalline lens and retina/choroid. Directly after sampling, all solid matrices were weighed (4 decimal precision) to determine the amount of each tissue collected.

Bilastine concentration was determined by LC-MS/MS.

Bilastine analyses were performed according to analytical methods:

- PKH/MOA/1022 for rabbit plasma,
- PKH/MOA/1042 for rabbit aqueous humor,
- PKH/MOA/1043 for rabbit vitreous humor,
- PKH/MOA/1028 for rabbit cornea homogenate,
- PKH/MOA/1041 for rabbit iris/ciliary body homogenate,
- PKH/MOA/1036 for rabbit conjunctiva homogenate,
- PKH/MOA/1032 for rabbit crystalline lens homogenate,
- PKH/MOA/1029 for rabbit retina/choroid homogenate,

which were previously developed and validated at Eurofins|ADME BIOANALYSES according to the Guidance for Industry - Bioanalytical Method Validation - FDA May 2018 and EMA Guideline - EMEA/CHMP/EWP/192217/2009 - 21 July 2011,

in the studies 18-014A to 18-014H.

The analytical methods involved protein precipitation for plasma and involved dilution for other matrices followed by LC-MS/MS analysis using F21201RR (Bilastine-d6) as internal standard.

The results showed that, 24 hours post-administration, significant concentrations of bilastine were found in conjunctiva (mean value: 388.45 ng/g), whereas the remainder eye tissue comprised less bilastine concentrations: cornea (mean value: 28.68 ng/g), iris/ciliary body (12.42 ng/g), retina/choroid (1.91 ng/g) and crystalline lens (0.12 ng/g).

## Claims

**1.** An aqueous ophthalmic pharmaceutical composition comprising:

a) at least 0.4% w/v but no more than 1.0% w/v of bilastine, or a pharmaceutically acceptable salt or solvate thereof, wherein the bilastine or salt or solvate thereof is completely dissolved in the aqueous ophthalmic pharmaceutical composition;
b) at least one β-cyclodextrin selected from the group consisting of unmodified β-cyclodextrin, $C_1$-$C_6$ alkyl-β-cyclodextrin, $C_1$-$C_6$ hydroxyalkyl β-cyclodextrin, $C_1$-$C_6$ carboxyalkyl-β-cyclodextrin, carbonyl-β-cyclodextrin, $C_2$-$C_6$ sulfoalkylether β-cyclodextrin and mixtures thereof; and
c) at least one pharmaceutically acceptable water-soluble gelling agent or an acceptable salt thereof, selected from the group consisting of hyaluronic acid, gellan gum and mixtures thereof;

and wherein the pH value of the composition is comprised between 4 and 9, both lower and upper limits of the range included.

**2.** The ophthalmic pharmaceutical composition according to claim 1, wherein the at least one β-cyclodextrin is selected from the group consisting of unmodified β-cyclodextrin, $C_1$-$C_6$ alkyl-β-cyclodextrin, hydroxyethyl-β-cyclodextrin, hydroxypropyl-β-cyclodextrin, 2-hydroxybutyl-β-cyclodextrin, $C_1$-$C_6$ carboxyalkyl-β-cyclodextrin, carbonyl-β-cyclodextrin, $C_2$-$C_6$ sulfoalkylether β-cyclodextrin and mixtures thereof.

**3.** The ophthalmic pharmaceutical composition according to any one of claims 1 or 2, comprising at least 0.6% w/v of bilastine or pharmaceutically acceptable salt or solvate thereof wherein the bilastine or salt or solvate thereof is completely dissolved in the aqueous ophthalmic pharmaceutical composition.

**4.** The ophthalmic pharmaceutical composition according to any one of claims 1 to 3, comprising:

a) at least 0.6% w/v but no more than 1.0% w/v of bilastine or a pharmaceutically acceptable salt or solvate thereof, wherein the bilastine or said salt or solvate thereof is completely dissolved in the aqueous ophthalmic pharmaceutical composition;
b) at least one β-cyclodextrin selected from the group consisting of unmodified β-cyclodextrin, $C_1$-$C_6$ alkyl-β-cyclodextrin, hydroxyethyl-β-cyclodextrin, hydroxypropyl-β-cyclodextrin, 2-hydroxybutyl-β-cyclodextrin, $C_1$-$C_6$ carboxyalkyl-β-cyclodextrin, carbonyl-β-cyclodextrin, $C_2$-$C_6$ sulfoalkylether β-cyclodextrin and mixtures thereof, wherein the concentration of said β-cyclodextrin is at least 5% w/v but no more than 15% w/v; and
c) hyaluronic acid or a pharmaceutically acceptable salt thereof, wherein the concentration of said hyaluronic acid or a pharmaceutically acceptable salt thereof is at least 0.05% w/v but no more than 1% w/v.

**5.** The ophthalmic pharmaceutical composition according to any one of claims 1 or 3, wherein the β-cyclodextrin is a $C_1$-$C_6$ hydroxyalkyl β-cyclodextrin selected from the group consisting of hydroxyethyl-β-cyclodextrin, hydroxypropyl-β-cyclodextrin and 2-hydroxybutyl-β-cyclodextrin.

**6.** The ophthalmic pharmaceutical composition according to any one of claims 1 to 5, wherein the hyaluronic acid or a pharmaceutically acceptable salt thereof, has a molecular weight no greater than 600000 Da.

**7.** The ophthalmic pharmaceutical composition according to any one of claims 1 to 6, wherein the pH is between 5 and 8, both lower and upper limits of the range included.

**8.** The ophthalmic pharmaceutical composition according to any one of claims 1 to 7, wherein the composition has an osmolality comprised between about 250 mOsm/kg and about 600 mOsm/kg.

9. The ophthalmic pharmaceutical composition according to any one of claims 1 to 8 further comprising a tonicity agent selected from the group consisting of glycerin, sorbitol, mannitol, erythriol, arabitol, xylitol, ribitol, galactitol, multitol, macrogol, lactitol, and mixtures thereof.

10. The ophthalmic pharmaceutical composition according to any one of claims 1 to 9, comprising:

a) at least 0.6% w/v but no more than 1.0% w/v of bilastine or a pharmaceutically acceptable salt or solvate thereof, wherein the bilastine or said salt or solvate thereof is completely dissolved in the aqueous ophthalmic pharmaceutical composition;
b) at least one $C_1$-$C_6$ hydroxyalkyl β-cyclodextrin, wherein the concentration of said β-cyclodextrin is at least 5% w/v but no more than 15% w/v;
c) hyaluronic acid or a pharmaceutically acceptable salt thereof, wherein the concentration of said hyaluronic acid or a pharmaceutically acceptable salt thereof is at least 0.05% w/v but no more than 1% w/v;
d) from 0.001% w/v to 15% w/v of at least one pharmaceutically acceptable water-soluble polymer, selected from the group consisting of an ether derivative of cellulose, polyethylene glycol, polyvinyl alcohol, and mixtures thereof; and
e) from 0.05% w/v to 5% w/v of at least one tonicity agent selected from the group consisting of glycerin, sorbitol, mannitol, erythriol, arabitol, xylitol, ribitol, galactitol, multitol, macrogol, lactitol, and mixtures thereof.

11. The ophthalmic pharmaceutical composition according to any one of claims 1 to 10, comprising:

a) at least 0.6% w/v but no more than 1.0% w/v of bilastine or a pharmaceutically acceptable salt or solvate thereof, wherein the bilastine or said salt or solvate thereof is completely dissolved in the aqueous ophthalmic pharmaceutical composition;
b) at least one $C_1$-$C_6$ hydroxyalkyl β-cyclodextrin, wherein the concentration of said β-cyclodextrin is at least 5% w/v but no more than 15% w/v;
c) hyaluronic acid or a pharmaceutically acceptable salt thereof, wherein the concentration of said hyaluronic acid or a pharmaceutically acceptable salt thereof is at least 0.05% w/v but no more than 1% w/v;
d) from 0.001% w/v to 15% w/v of an ether derivative of cellulose; and
e) from 0.05% w/v to 5% w/v of glycerin.

12. The ophthalmic pharmaceutical composition according to any one of claims 1 to 11, comprising:

a) at least 0.6% w/v but no more than 1.0% w/v of bilastine or a pharmaceutically acceptable salt or solvate thereof, wherein the bilastine or said salt or solvate thereof is completely dissolved in the aqueous ophthalmic pharmaceutical composition;
b) at least one $C_1$-$C_6$ hydroxyalkyl β-cyclodextrin, wherein the concentration of said β-cyclodextrin is at least 5% w/v but no more than 15% w/v;
c) hyaluronic acid or a pharmaceutically acceptable salt thereof, wherein the concentration of said hyaluronic acid or a pharmaceutically acceptable salt thereof is at least 0.05% w/v but no more than 1% w/v;
d) from 0.005% w/v to 0.1% w/v of methylcellulose; and
e) from 0.5% w/v to 2% w/v of glycerin.

13. The ophthalmic pharmaceutical composition according to any one of claims 1 to 12 **characterized in that** it is a once-daily ophthalmic pharmaceutical composition.

14. The aqueous ophthalmic pharmaceutical composition according to any one of claims 1 to 13 for use in the treatment and/or prevention of a disorder or disease susceptible to amelioration by antagonism of $H_1$ histamine receptor, selected from an ocular allergic disorder or an allergic disease.

15. The aqueous ophthalmic pharmaceutical composition for use according to claim 14 wherein the disorder or disease susceptible to amelioration by antagonism of $H_1$ histamine receptor is rhinitis, rhinoconjunctivitis, allergic conjunctivitis, vernal keratoconjunctivitis, atopic keratoconjunctivitis, giant papillary conjunctivitis, ocular irritation, itchiness, redness, tearing, chemosis, keratitis sicca, keratoconjunctivitis sicca or dysfunctional tear syndrome.

**Patentansprüche**

1. Eine wässrige ophthalmische pharmazeutische Zusammensetzung, umfassend:

   a) mindestens 0,4 % w/v, jedoch nicht mehr als 1.0 % w/v Bilastin oder ein pharmazeutisch verträgliches Salz oder Solvat davon, wobei das Bilastin oder Salz oder Solvat davon vollständig in der wässrigen ophthalmischen pharmazeutischen Zusammensetzung gelöst ist;
   b) mindestens ein β-Cyclodextrin ausgewählt aus der Gruppe bestehend aus unmodifiziertem β-Cyclodextrin, $C_1$-$C_6$-Alkyl-β-cyclodextrin, $C_1$-$C_6$-Hydroxyalkyl-β-cyclodextrin, $C_1$-$C_6$-Carboxyalkyl-β-cyclodextrin, Carbonyl-β-cyclodextrin, $C_2$-$C_6$-Sulfoalkylether-β-cyclodextrin und Gemischen davon; und
   c) mindestens ein pharmazeutisch verträgliches wasserlösliches Geliermittel oder ein akzeptables Salz davon, ausgewählt aus der Gruppe bestehend aus Hyaluronsäure, Gellangummi und Gemische davon;

   und wobei der pH-Wert der Zusammensetzung zwischen 4 und 9 liegt, wobei sowohl die unteren als auch die oberen Grenzen des Bereichs eingeschlossen sind.

2. Die ophthalmische pharmazeutische Zusammensetzung gemäß Anspruch 1, wobei das mindestens eine β-Cyclodextrin ausgewählt ist aus der Gruppe bestehend aus unmodifiziertem β-Cyclodextrin, $C_1$-$C_6$-Alkyl-β-cyclodextrin, Hydroxyethyl-β-cyclodextrin, Hydroxypropyl-β-cyclodextrin, 2-Hydroxybutyl-β-cyclodextrin, $C_1$-$C_6$-Carboxyalkyl-β-cyclodextrin, Carbonyl-β-cyclodextrin, $C_2$-$C_6$-Sulfoalkylether-β-cyclodextrin und Gemischen davon.

3. Die ophthalmische pharmazeutische Zusammensetzung gemäß irgendeinem der Ansprüche 1 oder 2, umfassend mindestens 0,6 % w/v Bilastin oder ein pharmazeutisch verträgliches Salz oder Solvat davon, wobei das Bilastin oder Salz oder Solvat davon vollständig in der wässrigen ophthalmischen pharmazeutischen Zusammensetzung gelöst ist.

4. Die ophthalmische pharmazeutische Zusammensetzung gemäß irgendeinem der Ansprüche 1 bis 3, umfassend:

   a) mindestens 0,6 % w/v, jedoch nicht mehr als 1.0 % w/v Bilastin oder ein pharmazeutisch verträgliches Salz oder Solvat davon, wobei das Bilastin oder Salz oder Solvat davon vollständig in der wässrigen ophthalmischen pharmazeutischen Zusammensetzung gelöst ist;
   b) mindestens ein β-Cyclodextrin ausgewählt aus der Gruppe bestehend aus unmodifiziertem β-Cyclodextrin, $C_1$-$C_6$-Alkyl-β-cyclodextrin, Hydroxyethyl-β-cyclodextrin, Hydroxypropyl-β-cyclodextrin, 2-Hydroxybutyl-β-cyclodextrin, $C_1$-$C_6$-Carboxyalkyl-β-cyclodextrin, Carbonyl-β-cyclodextrin, $C_2$-$C_6$-Sulfoalkylether-β-cyclodextrin und Gemischen davon, wobei die Konzentration von β-Cyclodextrin mindestens 5 % w/v, jedoch nicht mehr als 15 % w/v; und
   c) Hyaluronsäure oder ein pharmazeutisch verträgliches Salz davon, wobei die Konzentration der Hyaluronsäure oder eines pharmazeutisch verträglichen Salzes davon mindestens 0,05 % w/v, jedoch nicht mehr als 1 % w/v beträgt.

5. Die ophthalmische pharmazeutische Zusammensetzung gemäß irgendeinem der Ansprüche 1 bis 3, wobei das β-Cyclodextrin ein $C_1$-$C_6$-Hydroxyalkyl-β-cyclodextrin ist ausgewählt aus der Gruppe bestehend aus Hydroxyethyl-β-cyclodextrin, Hydroxypropyl-β-cyclodextrin und 2-Hydroxybutyl-β-cyclodextrin.

6. Die ophthalmische pharmazeutische Zusammensetzung gemäß irgendeinem der Ansprüche 1 bis 5, wobei die Hyaluronsäure oder ein pharmazeutisch verträgliches Salz davon ein Molekulargewicht von nicht mehr als 600000 Da aufweist.

7. Die ophthalmische pharmazeutische Zusammensetzung gemäß einem der Ansprüche 1 bis 6, wobei der pH zwischen 5 und 8 liegt, wobei sowohl die unteren als auch die oberen Grenzen des Bereichs eingeschlossen sind.

8. Die ophthalmische pharmazeutische Zusammensetzung gemäß einem der Ansprüche 1 bis 7, wobei die Zusammensetzung eine Osmolalität aufweist, die zwischen etwa 250 mOsm/kg und etwa 600 mOsm/kg liegt.

9. Die ophthalmische pharmazeutische Zusammensetzung gemäß einem der Ansprüche 1 bis 8, ferner umfassend ein Tonizitätsmittel, ausgewählt aus der Gruppe bestehend aus Glycerin, Sorbit, Mannit, Erythrit, Arabinit, Xylit, Ribit, Galactit, Multit, Makrogol, Lactit und Gemischen davon.

**10.** Die ophthalmische pharmazeutische Zusammensetzung gemäß einem der Ansprüche 1 bis 9, umfassend:

a) mindestens 0,6 % w/v, jedoch nicht mehr als 1,0 % w/v Bilastin oder ein pharmazeutisch verträgliches Salz oder Solvat davon, wobei das Bilastin oder das Salz oder Solvat davon vollständig in der wässrigen ophthalmischen pharmazeutischen Zusammensetzung gelöst ist;
b) mindestens ein $C_1$-$C_6$-Hydroxyalkyl-β-cyclodextrin, wobei die Konzentration des β-Cyclodextrins mindestens 5 % w/v, jedoch nicht mehr als 15 % w/v beträgt;
c) Hyaluronsäure oder ein pharmazeutisch verträgliches Salz davon, wobei die Konzentration der Hyaluronsäure oder eines pharmazeutisch verträglichen Salzes davon mindestens 0,05 % w/v, jedoch nicht mehr als 1 % w/v beträgt;
d) 0,001 % w/v bis 15 % w/v mindestens eines pharmazeutisch verträglichen wasserlöslichen Polymers, ausgewählt aus der Gruppe bestehend aus einem Etherderivat von Cellulose, Polyethylenglykol, Polyvinylalkohol und Gemischen davon; und
e) 0,05 % w/v bis 5 % w/v mindestens eines Tonizitätsmittels, ausgewählt aus der Gruppe bestehend aus Glycerin, Sorbit, Mannit, Erythrit, Arabinit, Xylit, Ribit, Galactit, Multit, Makrogol, Lactit und Gemische davon.

**11.** Die ophthalmische pharmazeutische Zusammensetzung gemäß einem der Ansprüche 1 bis 10 umfassend:

a) mindestens 0,6 % w/v, jedoch nicht mehr als 1,0 % w/v Bilastin oder ein pharmazeutisch verträgliches Salz oder Solvat davon, wobei das Bilastin oder das Salz oder Solvat davon vollständig in der wässrigen ophthalmischen pharmazeutischen Zusammensetzung gelöst ist;
b) mindestens ein $C_1$-$C_6$-Hydroxyalkyl-β-cyclodextrin, wobei die Konzentration des β-Cyclodextrins mindestens 5 % w/v, jedoch nicht mehr als 15 % w/v beträgt;
c) Hyaluronsäure oder ein pharmazeutisch verträgliches Salz davon, wobei die Konzentration der Hyaluronsäure oder eines pharmazeutisch verträglichen Salzes davon mindestens 0,05 % w/v, jedoch nicht mehr als 1 % w/v beträgt;
d) 0,001 % w/v bis 15 % w/v eines Etherderivats von Cellulose; und
e) 0,05 % w/v bis 5 % w/v Glycerin.

**12.** Die ophthalmische pharmazeutische Zusammensetzung gemäß einem der Ansprüche 1 bis 11, umfassend:

a) mindestens 0,6 % w/v, jedoch nicht mehr als 1,0 % w/v Bilastin oder ein pharmazeutisch verträgliches Salz oder Solvat davon, wobei das Bilastin oder das Salz oder Solvat davon vollständig in der wässrigen ophthalmischen pharmazeutischen Zusammensetzung gelöst ist;
b) mindestens ein $C_1$-$C_6$-Hydroxyalkyl-β-cyclodextrin, wobei die Konzentration des β-Cyclodextrins mindestens 5 % w/v, jedoch nicht mehr als 15 % w/v beträgt;
c) Hyaluronsäure oder ein pharmazeutisch verträgliches Salz davon, wobei die Konzentration der Hyaluronsäure oder eines pharmazeutisch verträglichen Salzes davon mindestens 0,05 % w/v, jedoch nicht mehr als 1 % w/v beträgt;
d) 0,005 % w/v bis 0,1 % w/v Methylcellulose; und
e) 0,5 % w/v bis 2 % w/v Glycerin.

**13.** Die ophthalmische pharmazeutische Zusammensetzung gemäß einem der Ansprüche 1 bis 12, **dadurch gekennzeichnet, dass** es sich um eine ophthalmische pharmazeutische Zusammensetzung handelt, die einmal täglich verabreicht wird.

**14.** Die wässrige ophthalmische pharmazeutische Zusammensetzung gemäß einem der Ansprüche 1 bis 13 zur Verwendung bei der Behandlung und/oder Vorbeugung einer Störung oder Krankheit, die für eine Verbesserung durch $H_1$-Histamin-Rezeptorantagonismus empfänglich ist, ausgewählt aus einer Augenallergie oder einer allergischen Erkrankung.

**15.** Die wässrige ophthalmische pharmazeutische Zusammensetzung zur Verwendung gemäß Anspruch 14, wobei die Störung oder Krankheit, die durch Antagonismus des $H_1$-Histamin-Rezeptors gebessert werden kann, Rhinitis, Rhinokonjunktivitis, allergische Konjunktivitis, frühlingshafte Keratokonjunktivitis, atopische Keratokonjunktivitis, Riesenpapillenkonjunktivitis, Augenreizung, Juckreiz, Rötung, Tränen, Chemosis, Keratitis sicca, Keratokonjunktivitis sicca oder dysfunktionelles Tränensyndrom ist.

## Revendications

**1.** Composition pharmaceutique aqueuse à usage ophtalmique comprenant :

a) au moins 0,4 % p/v mais pas plus de 1,0 % p/v de bilastine, ou d'un de ses sels ou solvates pharmaceutiquement acceptables, où la bilastine ou son sel ou solvate est complètement dissous dans la composition pharmaceutique aqueuse à usage ophtalmique ;
b) au moins une β-cyclodextrine choisie dans le groupe constitué par la β-cyclodextrine non modifiée, une (alkyle en $C_1$ à $C_6$)-β-cyclodextrine, une (hydroxyalkyle en $C_1$ à $C_6$)-β-cyclodextrine, une carboxy(alkyle en $C_1$ à $C_6$)-β-cyclodextrine, la carbonyl-β-cyclodextrine, une (sulfoalkyléther en $C_2$ à $C_6$)-β-cyclodextrine, et leurs mélanges ; et
c) au moins un agent gélifiant soluble dans l'eau pharmaceutiquement acceptable ou un de ses sels acceptables, choisi dans le groupe constitué par l'acide hyaluronique, la gomme gellane et leurs mélanges ;

et dans laquelle la valeur de pH de la composition est comprise entre 4 et 9, bornes comprises.

**2.** Composition pharmaceutique à usage ophtalmique selon la revendication 1, dans laquelle l'au moins une β-cyclodextrine est choisie dans le groupe constitué par la β-cyclodextrine non modifiée, une (alkyle en $C_1$ à $C_6$)-β-cyclodextrine, l'hydroxyéthyl-β-cyclodextrine, l'hydroxypropyl-β-cyclodextrine, la 2-hydroxybutyl-β-cyclodextrine, une carboxy(alkyle en $C_1$ à $C_6$)-β-cyclodextrine, la carbonyl-β-cyclodextrine, une (sulfoalkyléther en $C_2$ à $C_6$)-β-cyclodextrine, et leurs mélanges.

**3.** Composition pharmaceutique à usage ophtalmique selon l'une quelconque des revendications 1 ou 2, comprenant au moins 0,6 % p/v de bilastine ou d'un de ses sels ou solvates pharmaceutiquement acceptables, où la bilastine ou son sel ou solvate est complètement dissous dans la composition pharmaceutique aqueuse à usage ophtalmique.

**4.** Composition pharmaceutique à usage ophtalmique selon l'une quelconque des revendications 1 à 3, comprenant :

a) au moins 0,6 % p/v mais pas plus de 1,0 % p/v de bilastine ou d'un de ses sels ou solvates pharmaceutiquement acceptables, où la bilastine ou son sel ou solvate est complètement dissous dans la composition pharmaceutique aqueuse à usage ophtalmique ;
b) au moins une β-cyclodextrine choisie dans le groupe constitué par la β-cyclodextrine non modifiée, une (alkyle en $C_1$ à $C_6$)-β-cyclodextrine, l'hydroxyéthyl-β-cyclodextrine, l'hydroxypropyl-β-cyclodextrine, la 2-hydroxybutyl-β-cyclodextrine, une carboxy(alkyle en $C_1$ à $C_6$)-β-cyclodextrine, la carbonyl-β-cyclodextrine, une (sulfoalkyléther en $C_2$ à $C_6$)-β-cyclodextrine, et leurs mélanges, où la concentration de ladite β-cyclodextrine est d'au moins 5 % p/v mais non supérieure à 15 % p/v ; et
c) de l'acide hyaluronique ou un de ses sels pharmaceutiquement acceptables, où la concentration dudit acide hyaluronique ou d'un de ses sels pharmaceutiquement acceptables est d'au moins 0,05 % p/v mais non supérieure à 1 % p/v.

**5.** Composition pharmaceutique à usage ophtalmique selon l'une quelconque des revendications 1 ou 3, dans laquelle la β-cyclodextrine est une (hydroxyalkyle en $C_1$ à $C_6$)-β-cyclodextrine choisie dans le groupe constitué par l'hydroxyéthyl-β-cyclodextrine, l'hydroxypropyl-β-cyclodextrine et la 2-hydroxybutyl-β-cyclodextrine.

**6.** Composition pharmaceutique à usage ophtalmique selon l'une quelconque des revendications 1 à 5, dans laquelle l'acide hyaluronique ou un de ses sels pharmaceutiquement acceptables a un poids moléculaire non supérieur à 600 000 Da.

**7.** Composition pharmaceutique à usage ophtalmique selon l'une quelconque des revendications 1 à 6, dans laquelle le pH est compris entre 5 et 8, bornes comprises.

**8.** Composition pharmaceutique à usage ophtalmique selon l'une quelconque des revendications 1 à 7, où la composition a une osmolalité comprise entre environ 250 mOsm/kg et environ 600 mOsm/kg.

**9.** Composition pharmaceutique à usage ophtalmique selon l'une quelconque des revendications 1 à 8, comprenant en outre un agent de tonicité choisi dans le groupe constitué par le glycérol, le sorbitol, le mannitol, l'érythritol, l'arabitol, le xylitol, le ribitol, le galactitol, le multitol, le macrogol, le lactitol, et leurs mélanges.

**10.** Composition pharmaceutique à usage ophtalmique selon l'une quelconque des revendications 1 à 9, comprenant :

a) au moins 0,6 % p/v mais pas plus de 1,0 % p/v de bilastine ou d'un de ses sels ou solvates pharmaceutiquement acceptable, où la bilastine ou ledit sel ou solvate de celle-ci est complètement dissous dans la composition pharmaceutique aqueuse à usage ophtalmique ;
b) au moins une (hydroxyalkyle en $C_1$ à $C_6$)-β-cyclodextrine, où la concentration de ladite β-cyclodextrine est d'au moins 5 % p/v mais non supérieure à 15 % p/v ;
c) de l'acide hyaluronique ou un de ses sels pharmaceutiquement acceptables, où la concentration dudit acide hyaluronique ou d'un de ses sels pharmaceutiquement acceptables est d'au moins 0,05 % p/v mais non supérieure à 1 % p/v ;
d) de 0,001 % p/v à 15 % p/v d'au moins un polymère soluble dans l'eau pharmaceutiquement acceptable, choisi dans le groupe constitué par un dérivé éther de cellulose, le polyéthylèneglycol, le poly(alcool vinylique), et leurs mélanges ; et
e) de 0,05 % p/v à 5 % p/v d'au moins un agent de tonicité choisi dans le groupe constitué par le glycérol, le sorbitol, le mannitol, l'érythritol, l'arabitol, le xylitol, le ribitol, le galactitol, le multitol, le macrogol, le lactitol, et leurs mélanges.

**11.** Composition pharmaceutique à usage ophtalmique selon l'une quelconque des revendications 1 à 10, comprenant :

a) au moins 0,6 % p/v mais pas plus de 1,0 % p/v de bilastine ou d'un de ses sels ou solvates pharmaceutiquement acceptables, où la bilastine ou ledit sel ou solvate de celle-ci est complètement dissous dans la composition pharmaceutique aqueuse à usage ophtalmique ;
b) au moins une (hydroxyalkyle en $C_1$ à $C_6$)-β-cyclodextrine, où la concentration de ladite β-cyclodextrine est d'au moins 5 % p/v mais non supérieure à 15 % p/v ;
c) de l'acide hyaluronique ou un de ses sels pharmaceutiquement acceptables, où la concentration dudit acide hyaluronique ou d'un de ses sels pharmaceutiquement acceptables est d'au moins 0,05 % p/v mais non supérieure à 1 % p/v ;
d) de 0,001 % p/v à 15 % p/v d'un dérivé éther de cellulose ; et
e) de 0,05 % p/v à 5 % p/v de glycérol.

**12.** Composition pharmaceutique à usage ophtalmique selon l'une quelconque des revendications 1 à 11, comprenant :

a) au moins 0,6 % p/v mais pas plus de 1,0 % p/v de bilastine ou d'un de ses sels ou solvates pharmaceutiquement acceptables, où la bilastine ou ledit sel ou solvate de celle-ci est complètement dissous dans la composition pharmaceutique aqueuse à usage ophtalmique ;
b) au moins une (hydroxyalkyle en $C_1$ à $C_6$) -β-cyclodextrine, où la concentration de ladite β-cyclodextrine est d'au moins 5 % p/v mais non supérieure à 15 % p/v ;
c) de l'acide hyaluronique ou un de ses sels pharmaceutiquement acceptables, où la concentration dudit acide hyaluronique ou d'un de ses sels pharmaceutiquement acceptables est d'au moins 0,05 % p/v mais non supérieure à 1 % p/v ;
d) de 0,005 % p/v à 0,1 % p/v de méthylcellullose ; et
e) de 0,5 % p/v à 2 % p/v de glycérol.

**13.** Composition pharmaceutique à usage ophtalmique selon l'une quelconque des revendications 1 à 12, **caractérisée en ce qu'**il s'agit d'une composition pharmaceutique à usage ophtalmique devant être administrée une fois par jour.

**14.** Composition pharmaceutique à usage ophtalmique selon l'une quelconque des revendications 1 à 13, pour une utilisation dans le traitement et/ou la prévention d'un trouble ou d'une maladie susceptible d'être amélioré par un antagonisme du récepteur d'histamine $H_1$, choisi parmi un trouble allergique oculaire et une maladie allergique.

**15.** Composition pharmaceutique à usage ophtalmique pour une utilisation selon la revendication 14, dans laquelle le trouble ou la maladie susceptible d'être amélioré par un antagonisme du récepteur d'histamine $H_1$ est une rhinite, une rhinoconjonctivite, une conjonctivite allergique, une kératoconjonctivite printanière, une kératoconjonctivite atopique, une conjonctivite à papilles géantes, une irritation oculaire, une démangeaison, une rougeur, un larmoiement, un chémosis, une kératite sèche, une kératoconjonctivite sèche ou un syndrome du larmoiement dysfonctionnel.

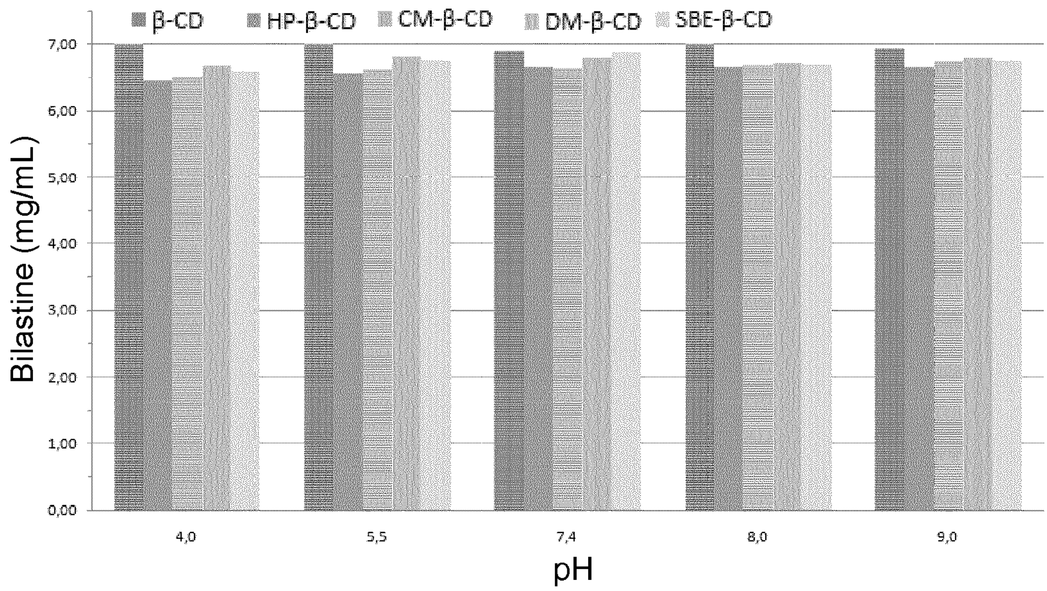
β-CD
HP-β-CD
CM-β-CD
DM-β-CD
SBE-β-CD
Bilastine (mg/mL)
7,00
6,00
5,00
4,00
3,00
2,00
1,00
0,00
4,0
5,5
7,4
8,0
9,0
pH

Fig. 1

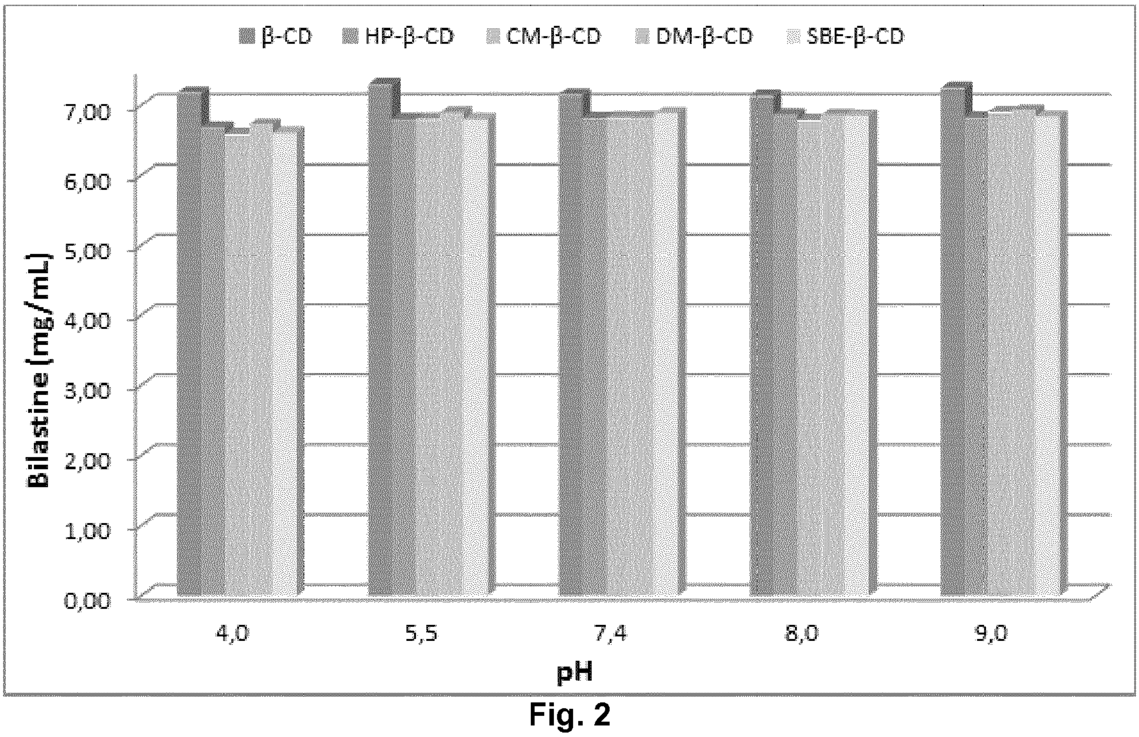
β-CD
HP-β-CD
CM-β-CD
DM-β-CD
SBE-β-CD
Bilastine (mg/mL)
7,00
6,00
5,00
4,00
3,00
2,00
1,00
0,00
4,0
5,5
7,4
8,0
9,0
pH

Fig. 2

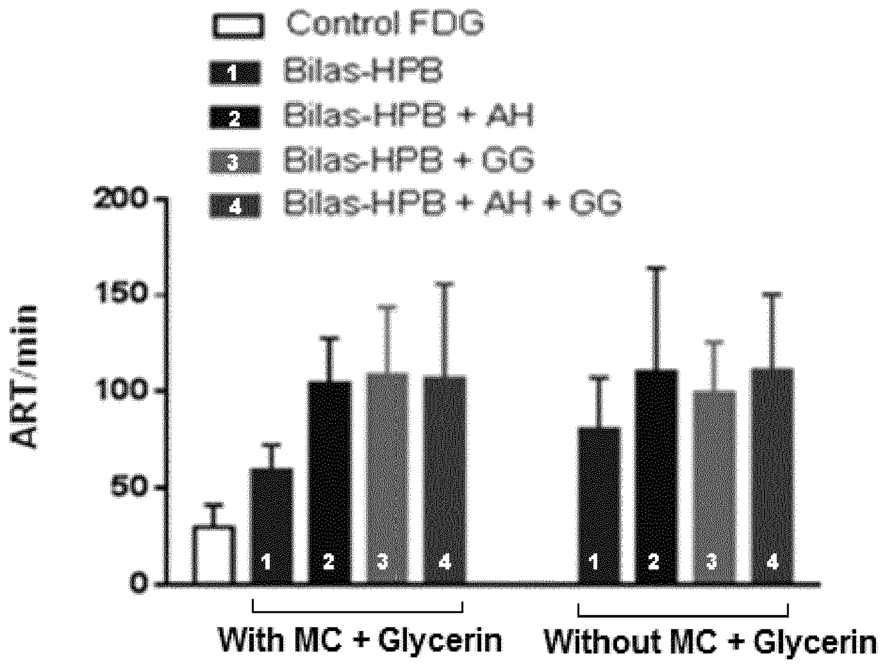
Control FDG
Bilas-HPB
Bilas-HPB + AH
Bilas-HPB + GG
Bilas-HPB + AH + GG
ART/min
200
150
100
50
0
1
2
3
4
1
2
3
4
With MC + Glycerin
Without MC + Glycerin

Fig. 3

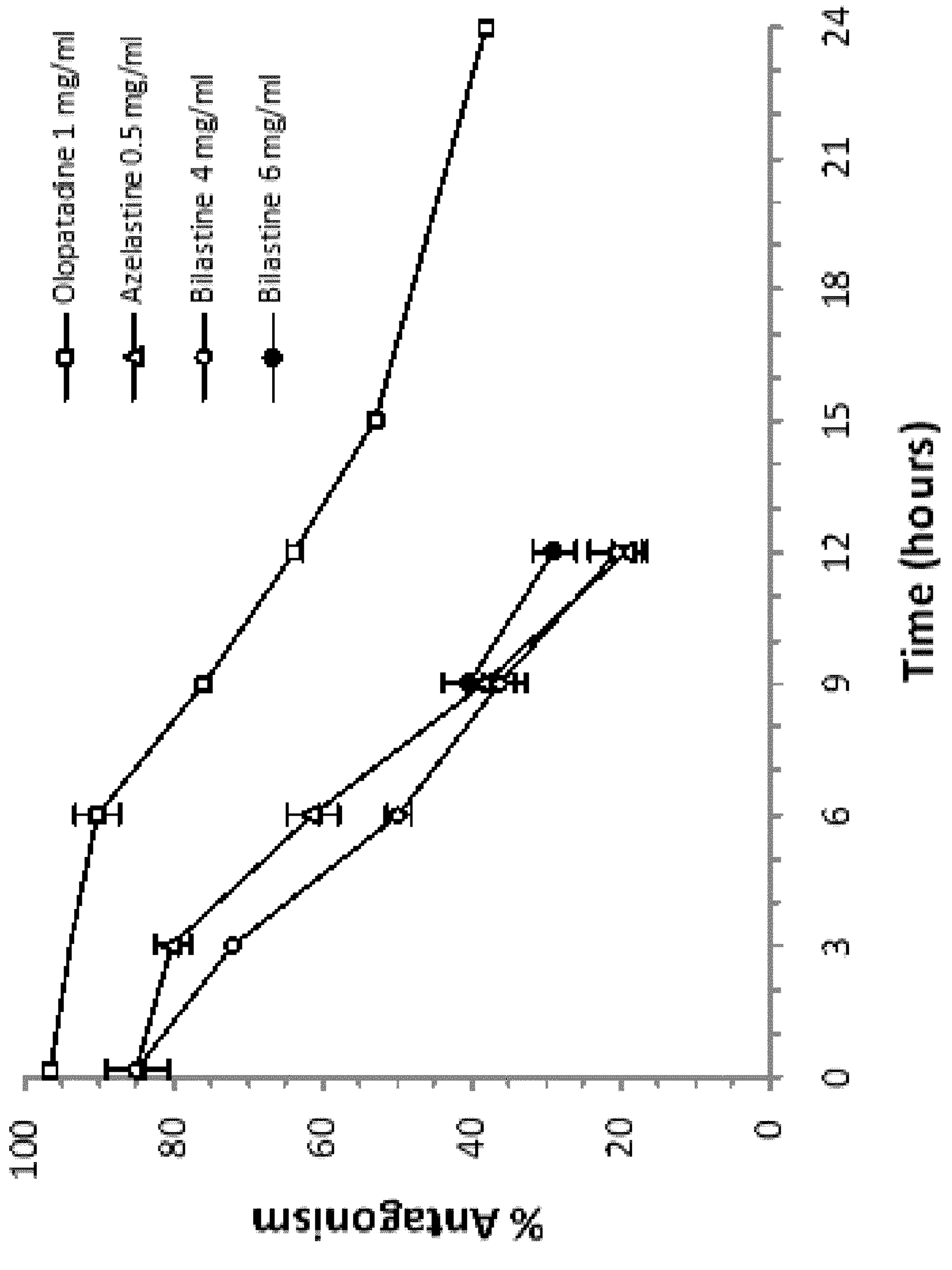
Olopatadine 1 mg/ml
Azelastine 0.5 mg/ml
Bilastine 4 mg/ml
Bilastine 6 mg/ml
% Antagonism
100
80
60
40
20
0
Time (hours)
0
3
6
9
12
15
18
21
24

Fig. 4

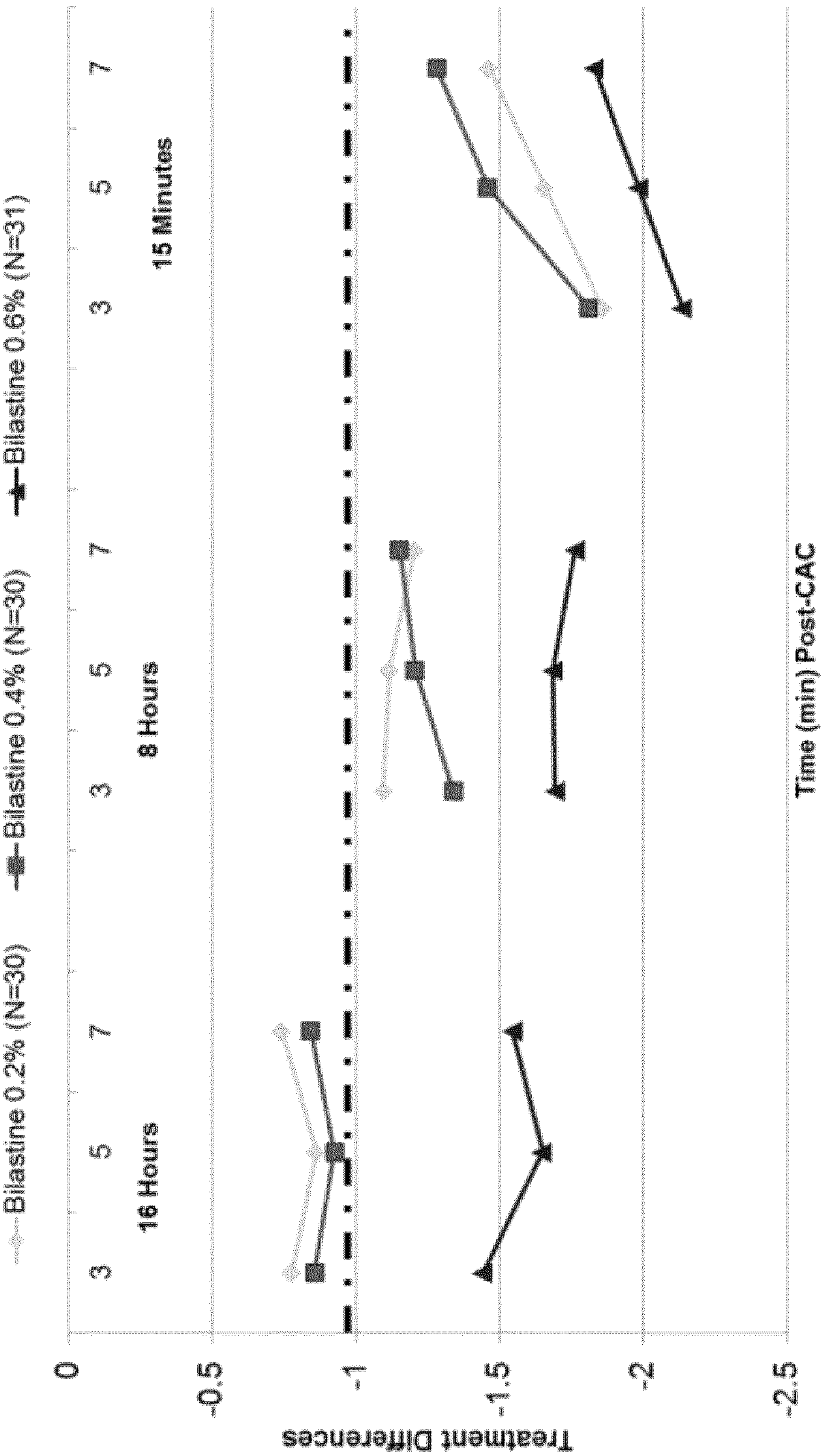
Bilastine 0.2% (N=30)
Bilastine 0.4% (N=30)
Bilastine 0.6% (N=31)
16 Hours
8 Hours
15 Minutes
3
5
7
3
5
7
3
5
7
0
-0.5
-1
-1.5
-2
-2.5
Treatment Differences
Time (min) Post-CAC

Fig. 5

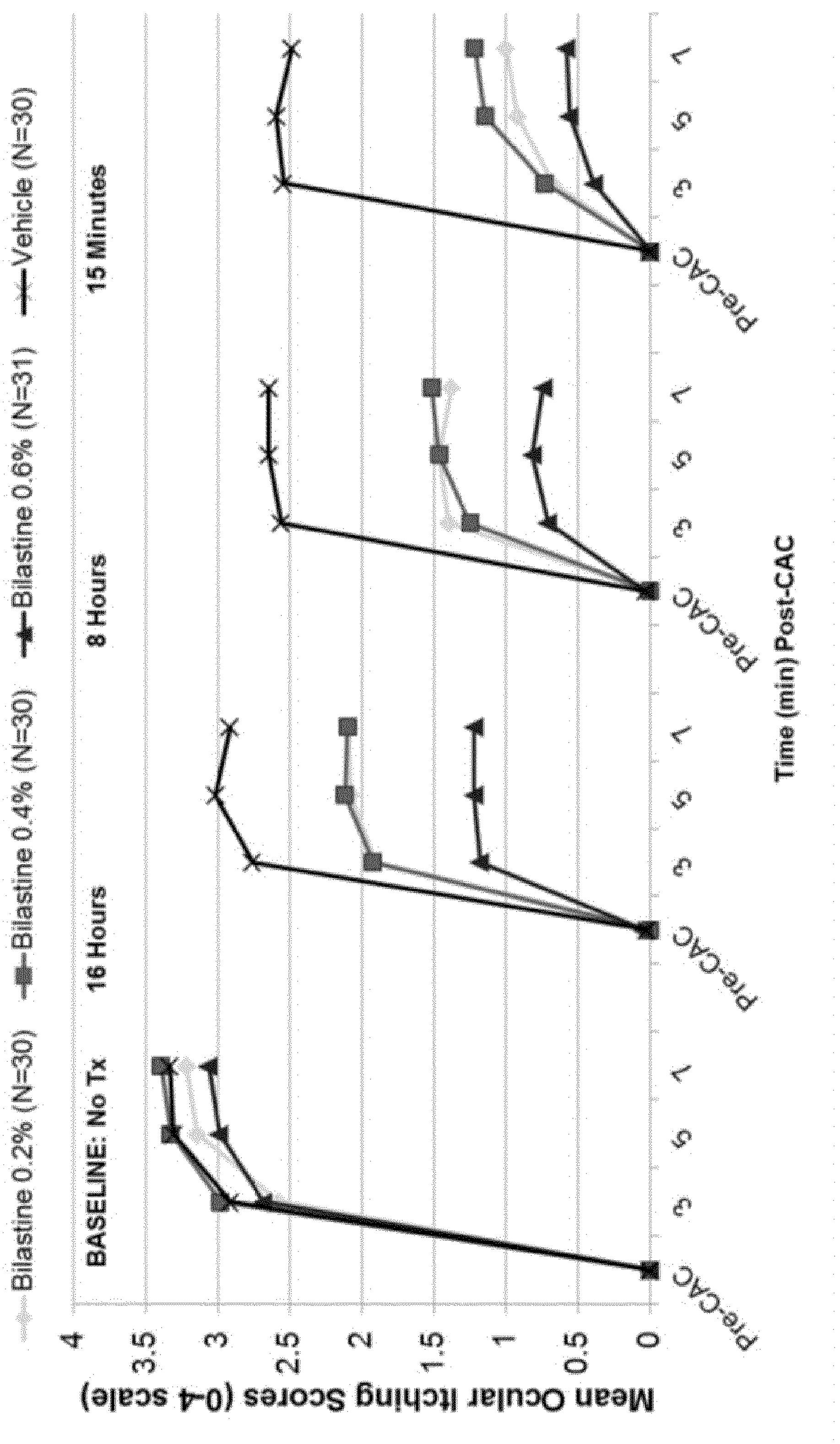
Bilastine 0.2% (N=30)
Bilastine 0.4% (N=30)
Bilastine 0.6% (N=31)
Vehicle (N=30)
BASELINE: No Tx
16 Hours
8 Hours
15 Minutes
Mean Ocular Itching Scores (0-4 scale)
4
3.5
3
2.5
2
1.5
1
0.5
0
Pre-CAC
3
5
7
Time (min) Post-CAC

Fig. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0818454 A1
- EP 0580541 A1
- EP 3040334 A1
- EP 2709610 B1
- WO 9413299 A1
- US 2007265247 A
- WO 03089425 A1
- WO 2007047253 A2
- WO 2009003199 A1
- CN 103784462 A
- WO 2007117971 A2
- US 2005239745 A1
- US 5419898 A


### Non-patent literature cited in the description


- **HORAK F et al.** *Inflammation Research,* 2010, vol. 59, 391-398
- **J BARTRA et al.** *J. Investig. Allergol. Clin. Immunol.,* 2011, vol. 21 (3), 24-33
- **BEAUREGARD C. et al.** *ARVO Annual Meeting Abstract,* May 2008
- **JOEL R. GARBOW et al.** *J Agric Food Chem.,* 2001, vol. 49 (4), 2053-2060
- **YUEJIANG LIU et al.** *AAPS PHARMSCITECH,* 2010, vol. 11 (2), 610-620
- **E.W. MARTIN.** Remington's Pharmaceutical Sciences. 2005
- **C. FAULÍ I TRILLO ; LUZÁN.** Tratado de Farmacia Galénica. 1993
- **BEAUREGARD C ; STEPHENS D ; ROBERTS L ; GAMACHE D ; YANNI J.** Duration of action of topical antiallergy drugs in a Guinea pig model of histamine-induced conjunctival vascular permeability. *J Ocul Pharmacol Ther.,* 2007, vol. 23, 315-20
- **VOGELSON CT ; ABELSON MB ; PASQUINE T ; STEPHENS DM ; GAMACHE DA ; GROSS RD ; ROBERTSON SM ; YANNI JM.** Preclinical and clinical antiallergic effect of olopatadine 0.2% solution 24 hours after topical ocular administration. *Allergy Asthma Proc.,* 2004, vol. 25, 69-75
- **TORKILDSEN G. ; NARVEKAR A. ; BERGMANN M.** Efficacy and safety of olopatadine hydrochloride 0.77% in patients with allergic conjunctivitis using a conjunctival allergen-challenge model. *Clinical Ophthalmology,* 2015, vol. 9, 1703-1713
- **C. BEAUREGARD ; D. J. STEPHENS ; S. T. MILLER ; L. ROBERTS ; D. A. GAMACHE ; J. M. YANNI.** ARVO Annual Meeting Abstract, Comparative Effects of Olopatadine, Bepotastine, and Bilastine. *Conjunctival Mast Cell Stabilization and Histamine-Induced Vascular Permeability,* May 2008, vol. 49 (13
- ADME BIOANALYSES according to the Guidance for Industry. Bioanalytical Method Validation. FDA, May 2018